(19) **Europäisches Patentamt / European Patent Office / Office européen des brevets**

(11) **EP 4 397 773 A1**

(12) **EUROPEAN PATENT APPLICATION**
published in accordance with Art. 153(4) EPC

(43) Date of publication:
**10.07.2024 Bulletin 2024/28**

(21) Application number: **22863369.9**

(22) Date of filing: **29.08.2022**

(51) International Patent Classification (IPC):
*C12Q 1/6869* (2018.01)      *G16B 20/20* (2019.01)
*G16B 20/30* (2019.01)      *G16B 30/10* (2019.01)

(52) Cooperative Patent Classification (CPC):
**C12Q 1/6869; G16B 20/20; G16B 20/30; G16B 30/10**

(86) International application number:
**PCT/CN2022/115447**

(87) International publication number:
**WO 2023/030233 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 30.08.2021   CN 202111002171
17.09.2021   CN 202111095132

(71) Applicant: **Guangzhou Burning Rock DX Co., Ltd.
Guangzhou, Guangdong 510300 (CN)**

(72) Inventors:
• LIU, Chenglin
**Guangzhou, Guangdong 510300 (CN)**
• ZHAO, Yu
**Guangzhou, Guangdong 510300 (CN)**

• KUANG, Ting
**Guangzhou, Guangdong 510300 (CN)**
• ZHANG, Zhihong
**Guangzhou, Guangdong 510300 (CN)**
• ZHANG, Zhen
**Guangzhou, Guangdong 510300 (CN)**
• ZHANG, Guangliang
**Guangzhou, Guangdong 510300 (CN)**
• ZHANG, Zhou
**Guangzhou, Guangdong 510300 (CN)**
• CHUAI, Shaokun
**Guangzhou, Guangdong 510300 (CN)**
• HAN, Yusheng
**Guangzhou, Guangdong 510300 (CN)**

(74) Representative: **Elzaburu S.L.P.
Edificio Torre de Cristal
Paseo de la Castellana 259 C, planta 28
28046 Madrid (ES)**

(54) **COPY NUMBER VARIATION DETECTION METHOD AND APPLICATION THEREOF**

(57)    Provided are a copy number variation detection method and an application thereof. Also provided is a copy number status analysis method, including: dividing a target region of a sample to be tested into several window regions, acquiring sequencing data of a control window region in a sample group to be tested, and determining the copy number status of a target gene of the sample to be tested based on the sequencing data of the control window region.

EP 4 397 773 A1

DRAWINGS

Figure 1A

Figure 1B

**Description**

**TECHNICAL FIELD**

The present application relates to the field of bioinformatics, specifically involving a copy number variations (CNVs) detection method and an application thereof.

**BACKGROUND**

Copy number variations (CNVs) are one of the common types of variations in the human genome. CNVs include two types of variations: amplification and deletion. Detection of gene copy number variations can be used to monitor the genomic status of subjects, as well as to discover associations between specific diseases and certain genomic variations. For example, copy number variations in genes can lead to a variety of common genetic diseases, such as the risk of hereditary breast cancer due to deletion of the BRCA1/2 genes; copy number variations can also affect the occurrence and development of tumors. For example, amplification of the HER2 gene is not only related to tumor development but is also an important clinical treatment monitoring and prognostic indicator, and a crucial biomarker for targeted therapy in cancer. Therefore, the detection of copy number variations plays a significant role in monitoring the genomic status of subjects, genome-wide association studies, prevention of genetic diseases, and precision treatment in cancer. For example, subjects carrying certain specific copy number variations may have a higher lifelong risk of developing diseases (such as cancer) compared to the general population. Thus, the method for detecting copy number variations can be used to screen subjects at higher risk, who can then receive individualized disease monitoring, thereby achieving early diagnosis and treatment.

Traditional methods for detecting copy number variations, such as ddPCR (droplet digital PCR), MLPA (Multiplex Ligation-dependent Probe Amplification), FISH (Fluorescent In Situ Hybridization), etc., can only detect the copy number status of one or a few genes at a time, or only specific gene copy numbers, and cannot perform a global analysis of the genome. The methods are characterized by low throughput and high cost. Currently, there are many methods based on high-throughput sequenceing technology for detecting copy number variations, but there are significant differences in the results of different detection methods, and the sensitivity and specificity of detection have certain limitations. On one hand, high-throughput sequencing technologies have certain batch effects and technical errors in the library construction and sequencing process. On the other hand, due to the complexity of tumor samples, there are great challenges in the stability of copy number detection results, making it difficult to detect copy number variations using high-throughput sequencing technology in the field of precision medicine. There is an urgent need in this field for an analytical method that can reduce batch effects, errors, and/or improve the stability of copy number detection results.

**SUMMARY**

The aim of the present application is to address the deficiencies in existing technologies by providing a method for detecting abnormalities in gene copy numbers. This method can at least reduce batch effects, errors, and/or improve the stability of copy number detection results, which is of great importance for determining copy number variation-related driving events and interpreting tumor genomic evolution information. The present application provides a copy number variation detection method and application thereof.

In one aspect, the present application provides a copy number status analysis method, the method includes dividing a target region of a sample to be tested into several window regions, acquiring sequencing data of a control window area within the sample group to be tested, and determining the copy number status of a target gene of the sample to be tested based on the sequencing data of the control window regions; optionally, the control window region includes window regions with low coverage fluctuation levels.

In one aspect, the present application provides a copy number status analysis device, including the following modules: a receiving module for acquiring sequencing data of a sample group to be tested; a determination module for determining the target gene of the sample to be tested; a judgment module for determining the copy number status of the target gene of the sample to be tested based on the sequencing data of the sample group to be tested.

In one aspect, the present application provides a storage medium, which records programs capable of executing the methods in the present application.

Those skilled in the art will readily perceive other aspects and advantages of the present application from the detailed description below. The detailed description only illustrates exemplary embodiments of the present application. As those skilled in the art will recognize, the content of the present application enables modifications to the disclosed specific embodiments without departing from the spirit and scope of the invention involved. Accordingly, the descriptions in the drawings and specification are merely exemplary and not intended to be limiting.

**BRIEF DESCRIPTION OF THE DRAWINGS**

The specific features of the invention involved in the present application are as shown in the attached claims. A better understanding of the characteristics and advantages of the invention involved in the present application can be obtained by referring to the exemplary embodiments and drawings described below. A brief description of the drawings is as follows:

Figures 1A-1B show the detection result graphs based on the method constructing a reference baseline and the method of the present application. Each box plot represents the distribution of BRCA1 gene exon copy number values for 30 samples. Group A and Group B represent probe captures of different batches, respectively. Figure 1A shows the distribution of BRCA1 gene exon copy numbers calculated based on the method constructing a reference baseline. Figure 1B shows the distribution of BRCA1 gene exon copy numbers calculated using the method of the present application.

Figures 2A-2B show the sample detection result graphs for the differences in NGS library construction methods based on the method constructing a reference baseline and the method of the present application. The horizontal axis: chromosome coordinates; the vertical axis: assessed copy number (CN) values. Figure 2A shows the copy number variation detection results based on the method constructing a reference baseline. Figure 2B shows the copy number variation detection results using the method of the present application. The boxes indicate detected copy number variations.

Figures 3A-3B show the sample detection results after selecting stable windows with different threshold settings. The horizontal axis: chromosome coordinates; the vertical axis: assessed copy number (CN) values. Figure 3A shows the copy number variation detection results with a threshold set to 0.05. Figure 3B shows the copy number variation detection results with a threshold set to 0.15. The boxes indicate detected copy number variations.

Figures 4A-4J show the detection results for 10 simulated positive copy number variation samples after constructing batch baselines. The horizontal axis: chromosome coordinates; the vertical axis: assessed copy number (CN) values. The boxes in the figures indicate detected copy number variations.

Figures 5A-5F show examples of the copy number distribution graphs for part of the detection results for simulated samples according to the present application.

Figures 6A-6C show examples of the copy number distribution graphs for part of the detection results for standard samples according to the present application.

Figures 7A-7C show examples of the copy number distribution graphs for part of the detection results for real samples according to the present application.

Figures 8A-8F show examples of the copy number distribution graphs for one standard sample using different baselines for detection results according to the present application.

**DETAILED DESCRIPTION OF THE EMBODIMET**

The implementation of the invention in the present application is illustrated by specific detailed examples, which can be easily understood by those skilled in the art from the content disclosed in this specification regarding other advantages and effects of the invention of the present application.

In the present application, the terms "second-generation gene sequencing", "high-throughput sequencing", or "next-generation sequencing" generally refer to the second generation of high-throughput sequencing technology and subsequent developments of even higher throughput sequencing methods. Next-generation sequencing platforms include, but are not limited to, existing sequencing platforms such as Illumina. With the continuous development of sequencing technology, those skilled in the art can understand that sequencing methods and devices using other approaches can also be adopted for this method. For example, next-generation sequencing can have advantages such as high sensitivity, large throughput, high sequencing depth, or low cost. Based on development history, influence, sequencing principles, and technology differences, the main types include: Massively Parallel Signature Sequencing (MPSS), Polony Sequencing, 454 pyrosequencing, Illumina (Solexa) sequencing, Ion semiconductor sequencing, DNA nano-ball sequencing, Complete Genomics DNA nano-array and combinatorial probe-anchor ligation sequencing, etc. Next-generation sequencing can make it possible to perform detailed and comprehensive analysis of a species' transcriptome and genome, hence it is also known as deep sequencing. For example, the method of the present application can also be applied to sanger sequencing, next-generation sequencing, third-generation sequencing, or single-molecule sequencing (SMS).

In the present application, the term "database" generally refers to an organized entity of related data, regardless of the representation of the data or the organized entity. For example, the organized entity of related data can take the form of tables, mappings, grids, groups, datagrams, files, documents, lists, or any other form. In the present application, the database can include any data collected and stored in a computer-accessible manner.

In the present application, the term "computational module" generally refers to a functional module used for computation. The computational module can calculate output values or conclusions or results based on input values, for example, the computational module can primarily be used to compute output values. The computational module can be tangible, such as the processor of an electronic computer, a computer or electronic device with a processor, or a computer network, or can be a program, command line, or software package stored on electronic media.

In the present application, the term "processing module" generally refers to a functional module used for data processing. The processing module can process input values into statistically significant data, for example, the processing module can be used for the classification of data from input values. The processing module can be tangible, such as electronic or magnetic media used for storing data, as well as the processor of an electronic computer, a computer or electronic device with a processor, or a computer network, or it can be a program, command line, or software package stored on electronic media.

In the present application, the term "judgment module" generally refers to a functional module used to obtain relevant judgment results. In the present application, the judgment module can calculate output values or conclusions or results based on input values, for example, the judgment module can primarily be used for obtaining conclusions or results. The judgment module can be tangible, such as the processor of an electronic computer, a computer or electronic device with a processor, or a computer network, or it can be a program, command line, or software package stored on electronic media.

In the present application, the term "sample acquisition module" generally refers to a functional module used to obtain the sample of the subject. For example, the sample acquisition module can include the reagents and/or instruments needed to obtain the sample (such as tissue, blood, saliva, pleural effusion, peritoneal effusion, cerebrospinal fluid, etc.). The sample acquisition module can include items such as blood collection needles, blood collection tubes, and/or blood sample transport boxes. For example, the device of the present application may not contain or contain one or more of the mentioned sample acquisition modules and may optionally have the function of outputting the measurements of the samples in the present application.

In the present application, the term "receiving module" generally refers to a functional module used to obtain the measurement values from the sample described. In the present application, the receiving module can input the samples in the present application (such as tissue, blood, saliva, pleural effusion, peritoneal effusion, cerebrospinal fluid, etc.). The receiving module can input the measurement values of the samples in the present application (such as tissue, blood, saliva, pleural effusion, peritoneal effusion, cerebrospinal fluid, etc.). The receiving module can detect the status of the sample. For example, the data receiving module can optionally perform the gene sequencing in the present application (such as next-generation sequencing). For example, the data receiving module can optionally include the reagents and/or instruments required for the gene sequencing. The data receiving module can optionally detect sequencing depth, sequencing read length count, or copy number.

In the present application, the term "copy number variation" generally refers to the amplification or deletion of copy numbers in a target region, target gene, or target region within a target gene. For example, the copy number variation analysis method provided in the present application can be used for therapeutic or diagnostic purposes. Alternatively, the copy number variation analysis method provided in the present application can be used for non-therapeutic or diagnostic purposes, such as determining the presence of copy number variations through sequencing results.

In the present application, the term "sliding window method" generally refers to a method of dividing a window region, for example, dividing a full-length region into multiple windows with the same or different window region lengths. Alternatively, the full-length region can be divided into multiple windows with the same or different step lengths. For example, the full-length region can be divided into multiple windows with the same window region length and the same step length.

In the present application, the term "quality-approved sample" generally refers to samples that meet quality control standards. For example, a quality-approved sample may refer to samples with an average sequencing depth, minimum sequencing depth, and/or coverage uniformity that meet the standards. For example, an average sequencing depth that qualifies may refer to samples with an average sequencing depth of approximately 100x or more. For example, a sample with minimum sequencing depth that qualifies may refer to sample with a minimum sequencing depth of approximately 30x or more. For example, a sample with coverage uniformity that qualifies may refer to sample where the percentage of bases that are greater than or equal to 20% of the sample's average sequencing depth constitutes approximately 90% or more of the total number of bases in the sample.

In the present application, the term "disqualified target region" generally refers to regions with low sequencing quality. For example, disqualified regions may not be suitable for analysis of copy number variations. Alternatively, disqualified regions may not be suitable for constructing references or baselines. In some cases, filtering out disqualified regions can improve the accuracy of the detection results; in other cases, not filtering out disqualified regions can also yield detection results with certain accuracy. For example, disqualified regions may refer to regions with low sequencing depth; for example, disqualified regions may refer to regions that vary greatly among different samples.

In the present application, the term "low capture efficiency region" generally refers to regions that are not easily captured by probes. For example, when a certain sequence combination exists in a region, it may be difficult to capture by nucleic acid probes. For example, low capture efficiency regions may refer to regions with low sequencing depth. For example, low capture efficiency regions may refer to regions where the sequencing read length counts is approximately 5 or lower.

In the present application, the term "unstable region" generally refers to regions where sequencing results vary greatly among different samples. For example, unstable regions can refer to regions where multiple sequencing results in the same sample vary greatly. For example, unstable regions can refer to regions where sequencing results vary greatly among samples in the same batch. For example, unstable regions can also refer to regions where sequencing results vary greatly among samples in different batches. For example, unstable regions can refer to regions where sequencing results vary greatly among different reference samples. For example, a method to determine unstable regions can be calculating the ratio of the standard deviation to the mean of sequencing depth for a certain region among different samples and determining whether this ratio exceeds a certain threshold, such as a threshold of 0.8, or adjusted by those skilled in the art based on actual sequencing conditions. In the present application, the term "sample to be tested" generally refers to samples that need to be tested to determine whether one or more gene regions on the sample exhibit copy number variations. For example, the sample to be tested or data thereof can be pre-stored in storage before testing.

In the present application, the term "human reference genome" generally refers to the human genome that can serve as a reference in gene sequencing. Information about the human reference genome can refer to UCSC (University of California, Santa Cruz). The human reference genome can have different versions, for example, the human reference genome can be hg19, GRCH37, or ensembl 75.

In the present application, the term "GC content" generally refers to the ratio of guanine (G) and cytosine (C) to the total nucleotides in a gene sequence (base sequence).

In the present application, the term "targeted sequencing panel" or "panel" generally refers to a set or group of detection targets. For example, in the process of sequencing, one or more target regions are captured and detected by designing one or more probes, and such one or more probes can constitute a targeted sequencing panel. For example, a targeted sequencing panel can be arbitrarily designed for target genes, target regions, or regions of interest, such as several exon regions. For example, probes can refer to oligonucleotides complementary to the target region's oligonucleotides or target nucleic acids used in research. The target region is the region aimed at when designing probes.

In the present application, the term "sequencing depth" commonly refers to the number of times a specific region (such as a specific gene, specific region, or specific base) is detected. Sequencing depth may refer to a sequence of bases detected through sequencing. For example, by mapping the sequencing to the human reference genome and optionally deduplicating, it is possible to determine and count the number of sequencing reads at specific genes, specific regions, or specific base positions, as a measure of sequencing depth. In some cases, sequencing depth can be related to the copy number status. For example, sequencing depth can be influenced by the copy number state.

In the present application, the term "sequencing data" generally refers to the data of short sequences obtained after sequencing. For example, sequencing data includes the base sequence of sequencing reads, the number of sequencing reads, etc.

In the present application, the term "sequencing bias" generally refers to the bias in sequencing data produced by different regions. For example, the special arrangement of sequences in a region or the base proportion can affect the count of sequencing reads for that region. For example, when a region has a higher or lower GC content, the count of sequencing reads for that region may deviate from that of regions with a GC content close to 50%.

In the present application, the term "degree of distribution similarity" can refer to the similarity in the distribution of two sets of data. For example, in the present application, the degree of distribution similarity can refer to the similarity in the count of sequencing reads between a reference sample group and a sample to be tested across one or more regions. In the present application, the term "statistical distance" can refer to the distance between the data values of two sets of data. For example, in the present application, statistical distance can refer to the statistical measure of the difference in the count of sequencing reads between a reference sample group and a sample to be tested across one or more regions. For example, the statistical distance can be calculated using Euclidean distance, Chebyshev distance, Mahalanobis distance, etc.

In the present application, the term "statistical value" can refer to analytical values calculated based on the data values of samples. For example, the statistical values in the present application can refer to mean, variance, standard deviation, median, mode, etc. Those skilled in the art may choose one or more statistical values for analyzing data based on actual situations.

In the present application, the term "probability distribution" generally refers to the distribution law of the values taken by a random variable. For example, depending on the type of random variable, the probability distribution can take different forms. For example, the normal distribution can serve as a probability distribution for a type of random variable. In the present application, the term "smoothing" generally refers to a data processing method that reduces the deviation among one or more differences in the present application. For example, smoothing may refer to fitting scatter data to a smooth line. For example, smoothing can be achieved through methods like local weighted regression to analyze and smooth the data. For example, after smoothing, it's possible to eliminate or reduce the inherent impact of a variable (e.g., GC content) on the sample sequencing data, thereby reducing the bias caused by this variable (e.g., GC content). For example, the smoothing can include obtaining the average of a certain number of differences in the present application. For example, the smoothing can include selecting data values corresponding to different lengths based on a certain region length, and calculating the differences among the data values. For example, the smoothing can include dividing the cumulative value of the differences within a certain length range by the region length to obtain a ratio. For example, the ratio can be considered the average difference for the differences within that length range. In the present application, the term "regression" generally refers to a statistical analysis method for the relationship between variables. For example, through regression analysis, the present application can establish the linear or nonlinear relationship between sample sequencing data and a certain variable (e.g., GC content). For example, the relationship between a sample's sequencing data and a variable (e.g., GC content) can be determined through local weighted regression, and this relationship can be used to adjust/correct the sample's sequencing data. For example, the correction in the present application can mean processing the sample's sequencing data based on its relationship with a variable to eliminate or reduce the bias this variable causes in the sequencing data.

In the present application, the term "local weighted regression" generally refers to a regression analysis method that introduces weights locally in the regression analysis between input variables and target variables. For example, local weighted regression can be conducted and processed through algorithms like loess(X-Y) based on Y for local weighted regression analysis of X.

In the present application, the term "denoising" generally refers to the removal or reduction of noise in data. For example, given that noise generally manifests as high-frequency signals, denoising can be achieved by extracting useful signals from noisy data using methods like transform analysis, principal component analysis, singular value decomposition, and/or Gaussian filtering.

In the present application, the term "cluster analysis" generally refers to a method of categorizing similar objects into different groups through classification, such that members within the same group share some similar attributes.

In the present application, the term "K-means clustering" generally refers to a method of cluster analysis. For example, K-means clustering can classify a set of data into several (K) categories based on K cluster centers, where each piece of data is closest to its nearest cluster center and minimizes the sum of distances.

In the present application, the term "transform analysis" generally refers to a method of analyzing data. For example, transform analysis can involve transforming the original distribution of data into a transformed domain distribution that is easier to solve or process for further analysis and processing. For example, the transform analysis can include discrete wavelet transforms.

In the present application, the term "discrete wavelet transform" generally refers to the discretization of the scale and translation of basic wavelets. For example, the discrete wavelet transform can serve as a method for denoising.

In the present application, the term "standardization" or "normalization" generally refers to a method of transforming data. For example, standardization can mean transforming data from different groups to a fixed range. For example, standardization can mean transforming data from different groups to the same median value. For example, standardization in the present application can refer to transforming the sequencing data of different samples to levels with similar median values.

In the present application, the term "significance testing" generally refers to a method for determining whether the difference between a sample and a hypothesized distribution is significant. For example, significance testing can determine whether copy number variation in a sample to be tested represents a significant difference.

In the present application, the term "normal distribution" generally refers to the probability distribution of a random variable. For example, the probability of occurrence of a random variable can be determined through the normal distribution and its density function. For example, the probability of the presence of copy number variation in a sample to be tested on target region can be confirmed based on the sequencing data of a reference sample group through the normal distribution.

In the present application, the term "Grubbs' test" generally refers to a method for identifying and/or removing outliers. For example, by determining whether a value fits within the overall distribution range, it can be determined whether the value is an outlier. In the present application, the term "T-test" generally refers to a statistical hypothesis test that follows a student's t-distribution. For example, a T-test can confirm whether copy number variation of a specific target gene in a sample to be tested is significant.

In the present application, the term "include" generally means including the features specifically mentioned, without excluding others.

In the present application, the term "approximately" generally refers to a range within 0.5%-10% above or below a specified value, for example, varying within 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

## DETAILED DESCRIPTION

In one aspect, the present application provides a copy number status analysis method, which can include acquiring sequencing data from a sample group to be tested; determining the target gene of the sample to be tested; and determining the copy number status of the target gene of the sample to be tested based on the sequencing data of the sample group to be tested.

In one aspect, the present application provides a copy number status analysis method, which can include the following steps:

(S1) dividing the region where the target gene is located into several window regions, and acquiring the sequencing data of the control window region from the sample group to be tested;

(S2) determining the copy number status of the target gene of the sample to be tested based on the sequencing data of the control window region.

In one aspect, the present application provides a copy number status analysis method, which can include the following steps:

(S1) dividing the region where the target gene is located into several window regions, and acquiring the sequencing data of the control window region from the sample group to be tested;

(S2) determining the copy number status of the target gene of the sample to be tested based on the sequencing data of the control window region. The window regions of the quality-approved samples are sorted from low to high according to the coverage fluctuation levels. The control window region can include the top 4 or more windows of coverage fluctuation level. The coverage fluctuation level can be determined based on the ratio of the median absolute deviation to the median of the sequencing data of the window regions of the quality-approved samples. Alternatively, the ratio of the median absolute deviation to the median of the sequencing data for all quality-approved samples in the control window region can be approximately 0.15 or smaller.

In one aspect, the present application provides a copy number status analysis method, which can include the following steps:

step (S 1-1): acquiring the sequencing data of the window regions of all samples in the sample group to be tested; step (S 1-2): acquiring quality-approved samples in the sample group to be tested, which may include samples that meet the criteria for average sequencing depth, minimum sequencing depth, and/or uniformity of coverage; step (S 1-3): normalizing the sequencing data of the window regions of all samples in the sample group to be tested;

(S2) determining the copy number status of the target gene of the sample to be tested based on the sequencing data of the control window region. The window regions of the quality-approved samples are sorted from low to high according to the coverage fluctuation levels.

The control window region can include the top 4 or more windows of coverage fluctuation level. The coverage fluctuation level can be determined based on the ratio of the median absolute deviation to the median of the sequencing data of the window regions of the quality-approved samples. Alternatively, the ratio of the median absolute deviation to the median of the sequencing data for all quality-approved samples in the control window region can be approximately 0.15 or smaller; step (S2-1): determining the normalization coefficient based on the sequencing data of the control window region; step (S2-2): determining the copy number of each window region of the sample to be tested based on the normalization coefficient; step (S2-3): determining the significance of copy number variation of the sample to be tested based on the sequencing data of each window region of the sample to be tested and the sequencing data of other samples in the sample group to be tested for the corresponding window region.

For example, the sequencing data can include sequencing depth. For example, the copy number status can include copy number amplification and/or deletion. For example, the copy number status can include exon copy number status.

For example, the sample group to be tested can include approximately 10 or more samples. For example, the sample group to be tested can include approximately 10 or more, approximately 12 or more, approximately 15 or more, approximately 20 or more, approximately 25 or more, approximately 50 or more, or approximately 100 or more samples. For example, the present application may not require a large number of samples in the same batch. For example, the sample group to be tested can include approximately 10 or fewer, approximately 12 or fewer, approximately 15 or fewer, approximately 20 or fewer, approximately 25 or fewer, or approximately 50 or fewer samples. For example, the method of copy number status analysis of the present application can have a high tolerance level for copy number variation in the samples to be tested. For example, samples containing approximately 30% copy number variation can be evaluated using the analysis method of the present application. For example, samples containing 10% or less, 15% or less, 20% or less, 25% or less, or 30% or less copy number variation can be evaluated using the analysis method of the present application. For example, the sample source of the present application can be any nucleic acid-containing sample, such as tissue, blood, saliva, pleural effusion, peritoneal effusion, cerebrospinal fluid, etc.

For example, the step (S1) of the method of the present application may also include step (S1-1): acquiring the sequencing data of the window regions of all samples in the sample group to be tested. For example, the gene sequencing of the present application can include any optional high-throughput sequencing method or module, device. For example, the sequencing can be selected from the following group: Solexa sequencing technology, 454 sequencing technology, SOLiD sequencing technology, Complete Genomics sequencing method, and semiconductor (Ion Torrent) sequencing technology and corresponding devices. For example, the step (S1-1) of the method of the present application can include dividing the region containing the target gene into the window regions through a sliding window method. For example, the step size of the sliding window method can be approximately 24 bases. For example, the length of the window region can be approximately 120 bases.

For example, the step (S 1-1) of the method of the present application can include acquiring the average sequencing depth after removing duplicate sequencing fragments for each of the window regions.

For example, the step (S1) of the method of the present application may also include step (S1-2): acquiring quality-approved samples from the sample group to be tested, where the quality-approved samples can include samples qualified in average sequencing depth, minimum sequencing depth, and/or coverage uniformity. For example, samples qualified in average sequencing depth can include samples with an average sequencing depth of approximately 100x or above. For example, samples qualified in minimum sequencing depth can include samples with a minimum sequencing depth of approximately 30x or above. For example, various quality thresholds can be adjusted according to the sequencing conditions.

For example, the coverage uniformity can be related to the sequencing depth of each base of the sample. For example, the coverage uniformity can be calculated by the percentage of bases whose sequencing depth is greater than or equal to 20% of the sample's average sequencing depth relative to the total number of bases in the sample. For example, the samples qualified in coverage uniformity can include samples with a coverage uniformity of approximately 90% or above. For example, the samples qualified in coverage uniformity can include samples with a coverage uniformity of approximately 90% or above, approximately 92% or above, approximately 95% or above, approximately 97% or above, or approximately 99% or above.

For example, the number of quality-approved samples in the sample group to be tested can be 10 or more.

For example, the step (S1) of the method of the present application may also include step (S 1-3): normalizing the sequencing data of the window regions of all samples in the sample group to be tested.

For example, the normalization can include normalizing the sequencing data for each window region of the sample based on the average sequencing depth of all window regions of the sample, and/or normalizing the sequencing data for each window region of the sample based on the GC content of each window region of the sample.

For example, the normalization can include dividing the sequencing data for each window region of the sample by the total of the sequencing data for all window regions of the sample, then multiplying by a factor. For example, the factor can be set based on the size of all regions. For example, the factor can be arbitrarily chosen as 1E+07. For example, the factor can be arbitrarily chosen as 1E+100, 1E+20, 1E+10, 1E+09, 1E+08, 1E+07, 1E+06, 1E+05, 1E+04, 1E+03, or 1E+02.

For example, the normalization can include normalizing the sequencing data for each window region of the sample based on GC content, through a regression method. For example, the regression can include local weighted regression.

For example, the control window region can include window regions with low fluctuation coverage levels.

For example, the coverage fluctuation levels can be determined based on the statistical values of the sequencing data of the window regions of the quality-approved samples. For example, the coverage fluctuation levels can be determined based on the deviation of the sequencing data of the window regions of the quality-approved samples. For example, the coverage fluctuation levels can be determined based on the median absolute deviation and/or median of the sequencing data of the window regions of the quality-approved samples. For example, the coverage fluctuation levels can be determined based on the ratio of the median absolute deviation to the median of the sequencing data of the window regions of the quality-approved samples.

For example, by sorting the window regions of the quality-approved samples according to the coverage fluctuation levels from low to high, the control window region can include the top 2 or more window regions with the lowest coverage fluctuation levels.

For example, by sorting the window regions of the quality-approved samples according to the coverage fluctuation levels from low to high, the control window region can include the top 4 or more window regions with the lowest coverage fluctuation levels.

For example, the ratio of the median absolute deviation to the median of the sequencing data for all quality-approved samples in the control window region can be approximately 0.15 or smaller. For example, the ratio of the median absolute deviation to the median of the sequencing data for all quality-approved samples in the control window region can be approximately 0.15 or smaller, approximately 0.14 or smaller, approximately 0.13 or smaller, approximately 0.12 or smaller, approximately 0.11 or smaller, approximately 0.10 or smaller, approximately 0.09 or smaller, approximately 0.08 or smaller, approximately 0.07 or smaller, approximately 0.06 or smaller, or approximately 0.05 or smaller. For example, the ratio of the median absolute deviation to the median of the sequencing data for all quality-approved samples in the control window region can be approximately 0.05 to approximately 0.15, approximately 0.07 to approximately 0.15, approximately 0.10 to approximately 0.15, approximately 0.12 to approximately 0.15, approximately 0.05 to approximately 0.12, approximately 0.07 to approximately 0.12, approximately 0.10 to approximately 0.12, approximately 0.05 to approximately 0.10, approximately 0.07 to approximately 0.10, or approximately 0.05 to approximately 0.07.

For example, the step (S2) in the present application can also include step (S2-1): determining a normalization coefficient based on the sequencing data of the control window region.

For example, the normalization coefficient can be determined by calculating the average value of the sequencing data of all quality-approved samples in the control window region. For example, before determining the normalization coefficient, it is possible to screen out the coverage level values of abnormal samples in the control window region. For example, the abnormal coverage level values can be the coverage level values of samples judged to be abnormal in each control window region through an outlier analysis method. For example, the outlier analysis method can include the Grubbs test. For example, each window can contain the coverage level values of quality-approved samples within that batch for that window, and then the Grubbs test can be used to determine whether the coverage levels contain outliers. If outliers are present, they can be removed. Then, for the remaining coverage level values, the Grubbs test can be repeated to determine the presence of outliers until no outliers are detected. For example, the process of removing outliers can stop when the number of remaining coverage level values is less than or equal to 60%, 50%, or 40% of the number of quality-approved samples; the remaining values can then be used to determine the normalization coefficient.

For example, the number of samples remaining after screening out the abnormal samples can be 40% or more, 70% or more, 80% or more, 90% or more, 95% or more, or 99% or more of the number of samples before screening.

For example, the step (S2) in the present application may also include step (S2-2): determining the copy number for each window region of the sample to be tested based on the normalization coefficient.

For example, the step (S2-2) in the present application can include determining the copy number for each window region of the sample to be tested by normalizing the sequencing data for each window region of the sample to be tested based on the normalization coefficient.

For example, the normalization method can include dividing window region of the sequencing data of the sample to be tested by the normalization coefficient for that window region, multiplied by ploidy. For example, for the male X chromosome, the ploidy can be 1. The ploidy can be adjusted according to specific conditions when the subject is polyploid. For example, the ploidy can be 2.

For example, the step (S2) in the present application may also include step (S2-3): determining the significance of copy number variation for the sample to be tested based on the sequencing data for each window region of the sample to be tested and the sequencing data of other samples in the sample group to be tested for the corresponding window region. For example, the step (S2-3) in the present application can include determining candidate regions for copy number variation based on the copy number for each window region of the sample to be tested.

For example, the candidate regions for copy number variation can be determined through a region segmentation method. For example, the region segmentation can include using a recursive binary splitting algorithm to determine the endpoints of the candidate region for copy number variation.

For example, the step (S2-3) in the present application can include determining the significance of the copy number variation based on the sequencing data of the window regions within the candidate region for copy number variation of the sample to be tested and the sequencing data of other samples in the sample group to be tested for the corresponding window regions. For example, the significance of the copy number variation can be determined through a significance testing method. For example, the significance testing can include a T-test.

In another aspect, the present application also provides a copy number status analysis device, which can include the following modules: a receiving module for acquiring the sequencing data of the sample group to be tested; a determination module for determining the target gene of the sample to be tested; a judgment module for determining the copy number status of the target gene of the sample to be tested based on the sequencing data of the sample group to be tested.

For example, the modules of the copy number status analysis device of the present application can be configured to perform the copy number status analysis method in the present application based on a program stored in the storage medium.

Copy Number Status Analysis Method

In one aspect, the present application provides a copy number status analysis method, which includes the following steps: (S1) acquiring sequencing data from the sample to be tested and/or multiple reference samples; (S2) dividing the reference samples into two or more reference sample groups; (S3) determining the reference sample group closest to the sample to be tested; (S4) based on the sequencing data of the reference sample group closest to the sample to be tested, determining the copy number status of the target gene of the sample to be tested.

In one aspect, the present application provides a copy number status analysis device, which can include the following modules: (M1) A receiving module, for acquiring sequencing data from the sample to be tested and/or multiple reference samples; (M2) A processing module, for dividing the reference samples into two or more reference sample groups; (M3) A computational module, for determining the reference sample group closest to the sample to be tested; (M4) A judgment module, for determining the copy number status of the target gene of the sample to be tested based on the sequencing data of the reference sample group closest to the sample to be tested.

In one aspect, the present application provides a copy number status analysis method, which can includes the following steps:

(S1) acquiring sequencing data from the sample to be tested and/or multiple reference samples; step (S1-1): acquiring the sequencing data of the sample to be tested and/or the reference samples through gene sequencing; step (S 1-2): correcting the sequencing data of the sample to be tested and/or reference samples;

(S2) dividing the reference samples into two or more reference sample groups; step (S2-1): grouping the reference samples; step (S2-2): confirming the statistical values of the sequencing data of the reference sample groups;

(S3) determining the reference sample group closest to the sample to be tested;

(S4) based on the sequencing data of the reference sample group closest to the sample to be tested, determining the copy number status of the target gene of the sample to be tested; step

(S4-1): determining the copy number $CN_i$ of the target gene in the target region i of the sample to be tested; step (S4-3): determining the copy number $CN_g$ of the sample to be tested on the target gene; step (S4-4): determining the probability of the presence of copy number variation in the target region of the sample to be tested; step (S4-5): determining the proportion of significant copy number amplification or deletion in the target gene of the sample to be tested as sigRatio; step (S4-6): determining the statistical test parameters for the presence of copy number variation in the target gene of the sample to be tested; the copy number status of the target gene of the sample to be tested is determined as follows: when $CN_g \geq CN_{thA}$, sigRatio$\geq$sigRatio$_{th}$, and $p_{ttest} \leq p_{th}$, it is confirmed that the target gene of the sample to be tested has undergone copy number amplification; when $CN_g \leq CN_{thD}$, sigRatio $\geq$sigRatio$_{th}$, and $p_{ttest} \leq p_{th}$, it is confirmed that the target gene of the sample to be tested has undergone copy number deletion; when $CN_{thA} < CN_g < CN_{thD}$, or sigRatio<sigRatio$_{th}$, or $p_{ttest} > p_{th}$, it is confirmed that the copy number of the target gene of the sample to be tested is normal, where $CN_{thA}$, $CN_{thD}$, sigRatio$_{th}$, and $p_{th}$ are each independently thresholds.

In one aspect, the present application provides a copy number status analysis device, which can include modules for implementing the copy number status analysis method of the present application.

In one aspect, the present application provides a copy number status analysis method, which can include the following steps:

(S1) acquiring sequencing data of the sample to be tested and/or sequencing data of one or more reference samples; step (S1-1): acquiring the sequencing data of the sample to be tested and/or the reference samples through gene sequencing; step (S 1-2): correcting the sequencing data of the sample to be tested and/or the reference samples;

(S2) dividing the reference samples into two or more reference sample groups; step (S2-1): grouping the reference samples; step (S2-2): confirming the statistical values of the sequencing data of the reference sample groups;

(S3) determining the reference sample group that is closest to the sample to be tested;

(S4) based on the sequencing data of the reference sample group closest to the sample to be tested, determining the copy number status of the target gene of the sample to be tested; step

(S4-1): determining the copy number $CN_i$ of the target gene of the sample to be tested on the target region i; step (S4-2): denoising the copy number on the target region of the sample to be tested; step (S4-3): determining the copy number $CN_g$ of the sample to be tested on the target gene; step (S4-4): determining the probability of the presence of copy number variation in the target region of the sample to be tested; step (S4-5): determining the proportion of significant copy number amplification or deletion present in the target gene of the sample to be tested, sigRatio; step (S4-6): determining the statistical test parameters for the presence of copy number variation in the target gene of the sample to be tested; Determining the copy number status of the target gene of the sample to be tested as follows: when $CN_g \geq CN_{thA}$, sigRatio$\geq$sigRatio$_{th}$, and $p_{ttest} \leq p_{th}$, it is confirmed that the target gene of the sample to be tested has undergone copy number amplification; when $CN_g \leq CN_{thD}$, $sigRatio \geq sigRatio_{th}$, and $p_{ttest} \leq p_{th}$, it is confirmed that the target gene of the sample to be tested has undergone copy number deletion; when $CN_{thA} < CN_g < CN_{thD}$, or $sigRatio < sigRatio_{th}$, or $p_{ttest} > p_{th}$, it is confirmed that the copy number of the target gene of the sample to be tested is normal, where $CN_{thA}$, $CN_{thD}$, $sigRatio_{th}$, and $p_{th}$ are each independently thresholds.

In one aspect, the present application provides a copy number status analysis device, which can include modules for implementing the copy number status analysis method of the present application.

For example, the sequencing data of the present application can include the count of sequencing read lengths. For example, the sequencing data of the present application can include the number of sequencing read length on the target gene or target region.

For example, the step (S1) or module (M1) of the present application can include step (S1-1) or module (M1-1): acquiring the sequencing data of the sample to be tested and/or the reference samples through gene sequencing. For example, the gene sequencing can include next-generation sequencing (NGS). For example, the gene sequencing of the present application can include any chosen high-throughput sequencing method or module, device. Sequencing can be selected from the group consisting of: Solexa sequencing technology, 454 sequencing technology, SOLiD sequencing technology, Complete Genomics sequencing method, and semiconductor (Ion Torrent) sequencing technology and their corresponding devices.

For example, the sample to be tested and/or the reference samples can include samples containing nucleic acids. For example, the sample source of the present application can be any sample containing nucleic acids, such as tissue, blood, saliva, pleural effusion, abdominal effusion, cerebrospinal fluid, etc.

For example, the step (S1-1) or module (M1-1) can include acquiring the sequencing data of each base in the target region of the sample to be tested and/or reference samples.

For example, the target region can include the region corresponding to the targeted sequencing panel sequence. The length of the target region can be approximately 20 to approximately 500 base pairs. For example, the length of the target region can be approximately 20 to approximately 500 base pairs, approximately 50 to approximately 500 base pairs, approximately 100 to approximately 500 base pairs, approximately 200 to approximately 500 base pairs, approximately 20 to approximately 200 base pairs, approximately 50 to approximately 200 base pairs, approximately 100 to approximately 200 base pairs, approximately 20 to approximately 100 base pairs, approximately 50 to approximately 100 base pairs, or approximately 20 to approximately 50 base pairs.

For example, the number of target regions can be at least approximately 100. For example, the number of target regions can be at least approximately 100, at least approximately 200, at least approximately 500, at least approximately 1000, or at least approximately 10000.

For example, the step (S1) or module (M1) can include step (S1-2) or module (M1-2): correcting the sequencing data of the sample to be tested and/or reference samples. For example, the method of the present application can also exclude step (S1-2) or only include part of step (S 1-2). For example, the device of the present application can also exclude module (M1-2) or only include part of module (M1-2). For example, the following sequence of steps in method step (S 1-2) can be arbitrary: normalizing the sequencing data of the sample to be tested and/or reference samples, smoothing the sequencing data of the sample to be tested and/or reference samples, and filtering out target regions with abnormal GC content. For example, the sequence of modules in device module (M1-2) can be arbitrary: normalizing the sequencing data of the sample to be tested and/or reference samples, smoothing the sequencing data of the sample to be tested and/or reference samples, and filtering out target regions with abnormal GC content.

For example, the step (S1-2) or module (M1-2) can include: normalizing or equalizing the sequencing data of the sample to be tested and/or reference samples. For example, the normalization or equalization can include dividing the sequencing data of the target region by the sum of the sequencing data of all target regions for the corresponding sample, then multiplying by a factor. For example, the factor can be set based on the size of all regions. For example, the factor can be arbitrarily chosen as 1E+07. For example, the factor can be arbitrarily chosen as 1E+100, 1E+20, 1E+10, 1E+09, 1E+08, 1E+07, 1E+06, 1E+05, 1E+04, 1E+03, or 1E+02.

For example, the step (S1-2) or module (M1-2) may include: smoothing the sequencing data of the sample to be tested and/or the reference sample. For example, the smoothing may include smoothing the sequencing data of the sample to be tested and/or reference sample based on sequencing bias, using regression methods or devices programmed for this process. For example, the regression may include locally weighted regression.

For example, the sequencing bias may include the number of probes covered on the target region.

For example, the sequencing bias may include the GC content of the target region.

For example, the step (S 1-2) or module (M1-2) may optionally include: screening out target regions with abnormal GC content.

For example, the target regions with abnormal GC content may include regions with a GC content of approximately 25% or lower and/or regions with a GC content of approximately 75% or higher.

For example, the step (S2) or module (M2) may include step (S2-1) or module (M2-1): grouping the reference samples. For example, the reference samples may come from the sample to be tested or from samples other than the sample to be tested. For example, part of the sample to be tested can be segmented out as reference samples. For example, the reference samples can be updated, such as adding the data of a new sample to an existing database after analyzing the sequencing data of new samples each time, and re-establishing the database.

For example, the grouping may include grouping the reference samples based on the sequencing data of the target region.

For example, the grouping may include grouping the reference samples through cluster analysis methods or devices programmed for this process.

For example, the methods of cluster analysis may include K-means clustering, hierarchical clustering, density clustering, grid clustering, probabilistic model clustering, or neural network model clustering, etc. The method or device programmed for cluster analysis may include any method or device programmed for clustering, classification, and grouping. For example, the number of reference samples may be approximately 30 or more. For example, the number of reference samples may be approximately 50 or more. For example, the number of reference samples may be approximately 30 or more, approximately 40 or more, approximately 50 or more, approximately 60 or more, approximately 70 or more, approximately 80 or more, approximately 90 or more, approximately 100 or more, approximately 200 or more, approximately 300 or more, approximately 400 or more, approximately 500 or more, or approximately 1000 or more.

For example, the grouping may include dividing into approximately 2 groups or more. For example, when the sequencing data of all reference samples are quite similar, only 1 group may the sequencing data be divided into. For example, the grouping includes dividing into approximately 2 or more, approximately 3 or more, approximately 4 or more, approximately 5 or more, approximately 6 or more, approximately 7 or more, approximately 8 or more, approximately 9 or more, approximately 10 or more, approximately 20 or more, approximately 30 or more, approximately 40 or more, approximately 50 or more, approximately 60 or more, approximately 70 or more, approximately 80 or more, approximately 90 or more, or approximately 100 or more.

For example, the number of reference samples in each group may be approximately 30 or more. For example, the number of reference samples in each group may be approximately 30 or more, approximately 40 or more, approximately 50 or more, approximately 60 or more, approximately 70 or more, approximately 80 or more, approximately 90 or more, approximately 100 or more, approximately 200 or more, approximately 300 or more, approximately 400 or more, approximately 500 or more, or approximately 1000 or more. For example, the step (S2) or module (M2) may include step (S2-2) or module (M2-2): confirming the statistical values of the sequencing data of the reference sample groups. For example, the statistical values of the sequencing data of the reference sample groups can serve as various candidate baselines. For example, confirming the statistical values can include calculating the mean and/or standard deviation of the reference samples in each group on the target region.

For example, the step (S2) or module (M2) may include step (S2-3) or module (M2-3): screening out disqualified target region from the reference samples. For example, the disqualified target region can include low capture efficiency regions and/or unstable regions. For example, the disqualified target region can include target regions with sequencing read length counts approximately 5 or lower. For example, disqualified target region can include target regions with sequencing read length counts approximately 30 or lower, approximately 20 or lower, approximately 10 or lower, approximately 5 or lower, approximately 4 or lower, approximately 3 or lower, approximately 2 or lower, approximately 1 or lower, or approximately 0 or lower.

For example, the disqualified target region can include target regions with a coefficient of variation approximately 0.8 or higher, where the coefficient of variation is the ratio of the standard deviation to the mean of the sequencing data of the reference samples in each group on the target region. For example, the disqualified target region can include target regions with a coefficient of variation approximately 0.8 or higher, approximately 0.9 or higher, or approximately 1.0 or higher. For example, the individual thresholds for low capture efficiency regions and/or unstable regions can be adjusted based on sequencing conditions. For example, the step (S3) or module (M3) may include confirming the similarity between the sample to be tested and the reference sample groups.

For example, confirming the similarity can include, based on the sequencing data of the reference sample groups and the sample to be tested on the target region, confirming the degree of distribution similarity between the reference sample groups and the sample to be tested.

For example, the similarity can include the approximation degree of the sequencing data of the reference sample groups and the sample to be tested on the target region.

For example, confirming the similarity can include confirming the degree of distribution similarity between the reference sample groups and the sample to be tested by calculating statistical distance, methods of similarity algorithms, or devices programmed for this process. For example, the statistical distance can include the statistical value of the difference in the sequencing data of the reference sample groups and the sample to be tested on the target region. For example, the statistical distance can include the statistical value of the absolute value of the difference in the sequencing data of the reference sample groups and the sample to be tested on the target region. For example, the statistical distance can include the statistical value of the p-th power of the absolute value of the difference in the sequencing data of the reference sample groups and the sample to be tested on the target region, where p is 1 or greater. For example, the statistical value can include the sum value. For example, the high similarity can include a short statistical distance between the reference sample group and the sample to be tested within the target region.

For example, the statistical distance can include the Minkowski distance. For example, the statistical distance can encompass Euclidean distance, Manhattan distance, Chebyshev distance, Minkowski distance (where p=1 corresponds to Manhattan distance; p=2 corresponds to Euclidean distance; and as p approaches infinity, becomes Chebyshev distance), etc. For example, the similarity algorithm may include cosine similarity, Pearson correlation coefficient, Spearman's rank correlation, log-likelihood similarity, cross-entropy, etc.

For example, the copy number state of the target gene of the sample to be tested can include the presence and/or quantity of copy number variation of the target gene of the sample to be tested.

For example, the copy number variation can include amplification and/or deletion.

For example, the step (S4) or module (M4) can include step (S4-1) or module (M4-1): determining the copy number $CN_i$ of the target gene in the target region i of the sample to be tested.

For example, determining the $CN_i$ can include dividing the mean sequencing data of the target gene in the target region of the sample to be tested by the mean sequencing data of the closest reference sample group in the corresponding target region, multiplied by the ploidy, to obtain $CN_i$.

For example, the ploidy can be 2. For example, for the male X chromosome, the ploidy can be 1. The ploidy can be adjusted according to specific conditions when the subject is polyploid.

For example, the step (S4) or module (M4) can include step (S4-2) or module (M4-2): denoising the copy number in the target region of the sample to be tested.

For example, denoising can include using transformation analysis, principal component analysis, singular value decomposition, and/or Gaussian filtering methods or devices recording such procedures to denoise the copy number in the target region of the sample to be tested.

For example, the denoising can include using discrete wavelet transform methods or devices recording such procedures to denoise the copy number in the target region of the sample to be tested. For example, the denoising can include using transformation analysis, principal component analysis, singular value decomposition, and/or Gaussian filtering methods or devices recording such procedures to denoise the copy number in the target region of the sample to be tested.

For example, the step (S4) or module (M4) can include step (S4-3) or module (M4-3): determining the copy number CNg of the sample to be tested on the target gene.

For example, the target gene can include genes where copy number variation is to be determined.

For example, the target gene can include genes selected from the following group: ABL1, ABL2, ABRAXAS1, ACVR1, ACVR1B, AKT1, AKT2, AKT3, ALK, ALOX12B, AMER1, APC, AR, ARAF, ARFRP1, ARID1A, ARID1B, ARID2, ARID5B, ASXL1, ASXL2, ASXL3, ATG5, ATM, ATR, ATRX, AURKA, AURKB, AXIN1, AXIN2, AXL, B2M, BAP1, BARD1, BBC3, BCL10, BCL2, BCL2L1, BCL2L11, BCL2L2, BCL6, BCOR, BCORL1, BIRC3, BLM, BMPR1A, BRAF, BRCA1, BRCA2, BRD4, BRD7, BRINP3, BRIP1, BTG1, BTG2, BTK, CALR, CARD11, CASP8, CBFB, CBL, CCND1, CCND2, CCND3, CCNE1, CD274, CD28, CD58, CD74, CD79A, CD79B, CDC73, CDH1, CDH18, CDK12, CDK4, CDK6, CDK8, CDKN1A, CDKN1B, CDKN1C, CDKN2A, CDKN2B, CDKN2C, CEBPA, CENPA, CHD1, CHD2, CHD4, CHD8, CHEK1, CHEK2, CIC, CIITA, CREBBP, CRKL, CRLF2, CRYBG1, CSF1R, CSF3R, CSMD1, CSMD3, CTCF, CTLA4, CTNNA1, CTNNB1, CUL3, CUL4A, CXCR4, CYLD, CYP17A1, CYP2D6, DAXX, DCUN1D1, DDR1, DDR2, DDX3X, DICER1, DIS3, DNAJB1, DNMT1, DNMT3A, DNMT3B, DOT1L, DPYD, DTX1, DUSP22, EED, EGFR, EIF1AX, EIF4E, EMSY, EP300, EPCAM, EPHA2, EPHA3, EPHA5, EPHA7, EPHB1, EPHB4, ERBB2, ERBB3, ERBB4, ERCC1, ERCC2, ERCC3, ERCC4, ERCC5, ERG, ERRFI1, ESR1, ETV4, ETV5, ETV6, EWSR1, EZH2, EZR, FANCA, FANCC, FANCD2, FANCE, FANCF, FANCG, FANCI, FANCL, FANCM, FAS, FAT1, FAT3, FBXW7, FGF10, FGF12, FGF14, FGF19, FGF23, FGF3, FGF4, FGF6, FGF7, FGFR1, FGFR2, FGFR3, FGFR4, FH, FLCN, FLT1, FLT3, FLT4, FOXA1, FOXL2, FOXO1, FOXO3, FOXP1, FRS2, FUBP1, FYN, GABRA6, GALNT12, GATA1, GATA2, GATA3, GATA4, GATA6, GEN1, GID4, GLI1, GNA11, GNA13, GNAQ, GNAS, GPS2, GREM1, GRIN2A, GRM3, GSK3B, H3F3A, H3F3B, H3F3C, HDAC1, HDAC2, HGF, HIST1H1C, HIST1H2BD, HIST1H3A, HIST1H3B, HIST1H3C, HIST1H3D, HIST1H3E, HIST1H3G, HIST1H3H, HIST1H3I, HIST1H3J, HIST2H3D, HIST3H3, HLA-A, HLA-B, HLA-C, HNF1A, HOXB13, HRAS, HSD3B1, HSP90AA1, ICOSLG, ID3, IDH1, IDH2, IFNGR1, IGF1, IGF1R, IGF2, IGHD, IGHJ, IGHV, IKBKE, IKZF1, IL10, IL7R, INHA, INHBA, INPP4A, INPP4B, INSR, IRF2, IRF4, IRS1, IRS2, ITK, ITPKB, JAK1, JAK2, JAK3, JUN, KAT6A, KDM5A, KDM5C, KDM6A, KDR, KEAP1, KEL, KIR2DL4, KIR3DL2, KIT, KLF4, KLHL6, KLRC1, KLRC2, KLRK1, KMT2A, KMT2C, KMT2D, KRAS, LATS1, LATS2, LMO1, LRP1B, LTK, LYN, MAF, MAGI2, MALT1, MAP2K1, MAP2K2, MAP2K4, MAP3K1, MAP3K13, MAP3K14, MAPK1, MAPK3, MAX, MCL1, MDC1, MDM2, MDM4, MED12, MEF2B, MEN1, MERTK, MET, MFHAS1, MGA, MIR21, MITF, MKNK1, MLH1, MLH3, MPL, MRE11, MSH2, MSH3, MSH6, MST1, MST1R, MTAP, MTOR, MUTYH, MYC, MYCL, MYCN, MYD88, MYOD1, NAV3, NBN, NCOA3, NCOR1, NCOR2, NEGR1, NF1, NF2, NFE2L2, NFKBIA, NKX2-1, NKX3-1, NOTCH1, NOTCH2, NOTCH3, NOTCH4, NPM1, NRAS, NRG1, NSD1, NSD2, NSD3, NT5C2, NTHL1, NTRK1, NTRK2, NTRK3, NUP93, NUTM1, P2RY8, PAK1, PAK3, PAK5, PALB2, PALLD, PARP1, PARP2, PARP3, PAX5, PBRM1, PCDH11X, PDCD1, PDCD1LG2, PDGFRA, PDGFRB, PDK1, PGR, PHOX2B, PIK3C2B, PIK3C2G, PIK3C3, PIK3CA, PIK3CB, PIK3CD, PIK3CG, PIK3R1, PIK3R2, PIK3R3, PIM1, PLCG2, PLK2, PMS1, PMS2, PNRC1, POLD1, POLE, POM121L12, PPARG, PPM1D, PPP2R1A, PPP2R2A, PPP6C, PRDM1, PREX2, PRKAR1A, PRKCI, PRKDC, PRKN, PTCH1, PTEN, PTPN11, PTPRD, PTPRO, PTPRS, PTPRT, QKI, RAB35, RAC1, RAD21, RAD50, RAD51, RAD51B, RAD51C, RAD51D, RAD52, RAD54L, RAF1, RARA, RASA1, RB1, RBM10, RECQL4, REL, RET, RHEB, RHOA, RICTOR, RIT1, RNF43, ROS1, RPA1, RPS6KA4, RPS6KB2, RPTOR, RSPO2, RUNX1, RUNX1T1, SDC4, SDHA, SDHAF2, SDHB, SDHC, SDHD, SETD2, SF3B 1, SGK1, SH2B3, SH2D1A, SHQ1, SLC34A2, SLIT2, SLX4, SMAD2, SMAD3, SMAD4, SMARCA4, SMARCB1, SMARCD1, SMO, SNCAIP, SOCS1, SOX10, SOX17, SOX2, SOX9, SPEN, SPI1, SPOP, SPTA1, SRC, SRSF2, STAG2, STAT3, STAT4, STAT5A, STAT5B, STAT6, STK11, STK40, SUFU, SYK, TAF1, TBX21, TBX3, TCF3, TCF7L2, TEK, TENT5C, TERC, TERT, TET1, TET2, TGFBR1, TGFBR2, TIPARP, TMEM127, TMPRSS2, TNFAIP3, TNFRSF14, TOP1, TOP2A, TP53, TP63, TP73, TRAF2, TRAF3, TRAF7, TRIM58, TRPC5, TSC1, TSC2, TSHR, TYRO3, U2AF1, UGT1A1, VEGFA, VEGFB, VEGFC, VHL, WISP3, WRN, WT1, XIAP, XPO1, XRCC2, XRCC3, YAP1, YES1, ZAP70, ZBTB16, ZBTB2, ZNF217, ZNF703 and ZNRF3. For example, the target gene can include genes selected from the following group: ALK (Transcript ID can be NM_004304.4), ERBB2 (Transcript ID can be NM_004448.3), EGFR (Transcript ID can be NM_005228.3), FGFR1 (Transcript ID can be NM_023110.2), FGFR2 (Transcript ID can be NM_000141.4), CDK4 (Transcript ID can be NM_000075.3), and MET (Transcript ID can be NM_000245.3).

For example, in the present application, samples are selected from the following group: tissue, blood, saliva, pleural effusion, peritoneal effusion, and cerebrospinal fluid.

For example, the step (S4-3) or module (M4-3) can include determining $CN_g$ based on the length of the exons of the target gene of the sample to be tested and the copy number $CN_i$ on the target region i of the sample to be tested.

For example, the step (S4-3) or module (M4-3) can include determining $CN_g$ based on the following formula,

$$CN_g = \frac{\sum_j^m \left( \frac{\sum_i^n (CN_i)}{n} * Len_j \right)}{\sum_j^m Len_j}$$

wherein i can represent the target region, j can represent the target exon, n can represent the number of target regions on target exon j, m can represent the number of target exons, $CN_i$ can represent the copy number of target region i, $Len_j$ can represent the length of target exon j.

For example, the step (S4) or module (M4) can include step (S4-4) or module (M4-4): determining the probability of the presence of copy number variation in the target region of the sample to be tested.

For example, the probability of the presence of copy number variation can include the probability ($p_a$) of amplification and/or the probability ($p_d$) of deletion occurring in the target region of the sample to be tested.

For example, the step (S4-4) or module (M4-4) can include, based on the sequencing data of the sample to be tested in the target region i, as well as the mean and standard deviation of the sequencing data of the closest reference sample group in the corresponding target region, confirming the probability of the presence of copy number variation through methods of probability distribution or devices programmed for this process.

For example, the probability distribution can include normal probability distribution. For example, the probability distribution can include any common probability distribution. For example, the probability distribution can include any discrete probability distribution. For example, the probability distribution can include any continuous probability distribution. For example, the step (S4) or module (M4) can include step (S4-5) or module (M4-5): determining the proportion of significant copy number amplification or deletion present in the target gene of the sample to be tested, sigRatio.

For example, the step (S4-5) or module (M4-5) can include dividing the number of target regions with significant copy number variation occurring on the target gene by the total number of target regions on the target gene, to obtain sigRatio.

For example, the target regions exhibiting significant copy number variations may include the target regions where the proportion of copy number variation is approximately 30% or higher. For example, the target regions exhibiting significant copy number variations may include the target regions where the proportion of copy number variation is approximately 30% or higher, approximately 40% or higher, approximately 50% or higher, approximately 60% or higher, approximately 70% or higher, approximately 80% or higher, approximately 90% or higher, approximately 95% or higher, or approximately 99% or higher.

For example, the step (S4) or module (M4) may include step (S4-6) or module (M4-6): determining the statistical test parameter for the presence of copy number variations in the target gene of the sample to be tested.

The statistical test parameter may include a p-value determined through significance testing. The significance test may include a T-test. For example, the significance test may be any significance testing method, and significance testing method modified according to the actual situation.

For example, the step (S4-6) or module (M4-6) may include, based on the number of target regions on the target gene of the sample to be tested, the sequencing data of each target region on the target gene of the sample to be tested, the standard deviation of the sequencing data for each target region on the target gene of the sample to be tested, and the mean and standard deviation of the sequencing data for the target regions on the corresponding target gene in the closest reference sample group to the sample to be tested, confirming the p-value ($p_{ttest}$) through a T-test method or a device that records program thereof.

For example, the step (S4) or module (M4) can determine the copy number status of the target gene of the sample to be tested by the following:

When $CN_g \geq CN_{thA}$, $sigRatio \geq sigRatio_{th}$, and $p_{ttest} \leq p_{th}$, it is confirmed that the target gene of the sample to be tested has undergone copy number amplification;

When $CN_g \leq CN_{thD}$, $sigRatio \geq sigRatio_{th}$, and $p_{ttest} \leq p_{th}$, it is confirmed that the target gene of the sample to be tested has undergone copy number deletion;

When $CN_{thA} < CN_g < CN_{thD}$, or $sigRatio < sigRatio_{th}$, or $p_{ttest} > p_{th}$, it is confirmed that the copy number of the target gene of the sample to be tested is normal, where $CN_{thA}$, $CN_{thD}$, $sigRatio_{th}$, and $p_{th}$ are independently thresholds.

For example, $CN_{thA}$ can be approximately 2.25 to approximately 4. For example, $CN_{thA}$ can be approximately 2.25, approximately 2.50, approximately 2.75, approximately 3.00, approximately 3.25, approximately 3.50, approximately 3.75, or approximately 4.00.

For example, $CN_{thD}$ can be approximately 1.0 to approximately 1.75. For example, $CN_{thD}$ can be approximately 0.25, approximately 0.50, approximately 0.75, approximately 1.00, approximately 1.25, approximately 1.50, approximately 1.75.

For example, $sigRatio_{th}$ can be approximately 0.3 to approximately 1. For example, $sigRatio_{th}$ can be approximately 0.3, approximately 0.4, approximately 0.5, approximately 0.6, approximately 0.7, approximately 0.8, approximately 0.9, or approximately 1.0.

For example, $p_{th}$ can be approximately 0.05 to approximately 0.00001. For example, $p_{th}$ can be approximately 0.05, approximately 0.01, approximately 0.001, approximately 0.0001, approximately 0.00001, approximately 0.000001, or approximately 0.0000001.

Database Establishment

In one aspect, the present application provides a database establishment method, which can include acquiring sequencing data from multiple reference samples, as well as dividing the reference samples into two or more groups of reference samples.

For example, the database establishment method can include: (S1) acquiring sequencing data from a sample to be tested and/or sequencing data from multiple reference samples; (S2) dividing the reference samples into two or more groups of reference samples.

In one aspect, the present application provides a database establishment device, which can include the following modules: a receiving module, for acquiring sequencing data from a sample to be tested and/or sequencing data from multiple reference samples; a processing module, for dividing the reference samples into two or more groups of reference samples. For example, the database establishment device can include: (M1) a receiving module, for acquiring sequencing data from a sample to be tested and/or sequencing data from multiple reference samples; (M2) a processing module, for dividing the reference samples into two or more groups of reference samples.

In one aspect, the present application provides a database establishment method, which can include the following steps:

(S1) acquiring sequencing data from a sample to be tested and/or sequencing data from multiple reference samples; step (S1-1): acquiring the sequencing data of the sample to be tested and/or the reference samples through gene sequencing; step (S 1-2): correcting the sequencing data of the sample to be tested and/or the reference samples; (S2) dividing the reference samples into two or more groups of reference samples; step (S2-1): grouping the reference samples; step (S2-2): confirming the statistical values of the sequencing data of the groups of reference samples.

In one aspect, the present application provides a database establishment device, which can include modules for implementing the database establishment method of the present application.

In one aspect, the present application provides a database establishment method, which can include the following steps:

(S1) acquiring sequencing data from a sample to be tested and/or sequencing data from multiple reference samples;

step (S1-1): acquiring the sequencing data of the sample to be tested and/or the reference samples through gene sequencing; step (S 1-2): correcting the sequencing data of the sample to be tested and/or the reference samples; (S2) dividing the reference samples into two or more groups of reference samples; step (S2-1): grouping the reference samples; step (S2-2): confirming the statistical values of the sequencing data of the groups of reference samples.

In one aspect, the present application provides a database establishment device, which can include modules for implementing the database establishment method of the present application.

In another aspect, the present application provides a database establishment device, which can include the following modules: (M1) a receiving module, for acquiring sequencing data from a sample to be tested and/or sequencing data from multiple reference samples; (M2) a processing module, for dividing the reference samples into two or more groups of reference samples.

Copy Number Status Analysis Method

In one aspect, the present application provides a copy number status analysis method based on the information from an existing database, which can include determining the reference sample group closest to the sample to be tested and determining the copy number status of the target gene of the sample to be tested based on the sequencing data from the closest reference sample group.

For example, the copy number status analysis method can include: (S3) determining the reference sample group closest to the sample to be tested; (S4) determining the copy number status of the target gene of the sample to be tested based on the sequencing data from the closest reference sample group.

In one aspect, the present application provides a copy number status analysis device, which can include the following modules: a computational module, for determining the reference sample group closest to the sample to be tested; a judgment module, for determining the copy number status of the target gene of the sample to be tested based on the sequencing data from the closest reference sample group.

For example, the copy number status analysis device can include: (M3) a computational module, for determining the reference sample group closest to the sample to be tested; (M4) a judgment module, for determining the copy number status of the target gene of the sample to be tested based on the sequencing data from the closest reference sample group.

In one aspect, the present application provides a copy number status analysis method, which can include the following steps:

(S3) determining the reference sample group closest to the sample to be tested;
(S4) determining the copy number status of the target gene of the sample to be tested based on the sequencing data from the closest reference sample group; step (S4-1) determining the copy number $CN_i$ of the target gene in the target region i; step (S4-3) determining the overall copy number $CN_g$ of the sample on the target gene; step (S4-4) determining the probability of the presence of copy number variation in the target region; step (S4-5) determining the ratio of significant copy number amplification or deletion presence sigRatio on the target gene of the sample to be tested; step (S4-6) determining the statistical test parameters for the presence of copy number variation on the target gene. The copy number status of the target gene of the sample to be tested is determined as follows: if $CN_g \geq CN_{thA}$, $sigRatio \geq sigRatio_{th}$, and $p_{ttest} \leq p_{th}$, it is confirmed that the target gene of the sample to be tested has undergone copy number amplification; if $CN_g \leq CN_{thD}$, $sigRatio \geq sigRatio_{th}$, and $p_{ttest} \leq p_{th}$, it is confirmed that the target gene of the sample to be tested has undergone copy number deletion; if $CN_{thA} < CN_g < CN_{thD}$, or $sigRatio < sigRatio_{th}$, or $p_{ttest} > p_{th}$, it is confirmed that the copy number of the target gene of the sample to be tested is normal, wherein $CN_{thA}$, $CN_{thD}$, $sigRatio_{th}$, and $p_{th}$ are each independently thresholds.

In one aspect, the present application provides a copy number status analysis device, which can include modules for implementing the copy number status analysis method of the present application.

In one aspect, the present application provides a copy number status analysis method, which can include the following steps:

(S3) determining the reference sample group closest to the sample to be tested;
(S4) based on the sequencing data of the reference sample group closest to the sample to be tested, determining the copy number status of the target gene of the sample to be tested; step (S4-1): determining the copy number $CN_i$ of the target gene of the sample to be tested in the target region i; step (S4-2): denoise the copy number on the target region of the sample to be tested; step (S4-3): determining the copy number $CN_g$ of the sample to be tested on the target gene; step (S4-4): determining the probability of the presence of copy number variation in the target region of the sample to be tested; step (S4-5): determining the proportion of significant copy number amplification or deletion on the target gene of the sample to be tested, sigRatio; step (S4-6): determining the statistical test parameters for the presence of copy number variation on the target gene of the sample to be tested; the copy number status of the target gene of the sample to be tested is determined by the following: when $CN_g \geq CN_{thA}$, $sigRatio \geq sigRatio_{th}$, and $p_{ttest} \leq p_{th}$, it is confirmed that the target gene of the sample to be tested has undergone copy number amplification; when $CN_g \leq CN_{thD}$, $sigRatio \geq sigRatio_{th}$, and $p_{ttest} \leq p_{th}$, it is confirmed that the target gene of the sample to be tested has undergone copy number deletion; when $CN_{thA} < CN_g < CN_{thD}$, or $sigRatio < sigRatio_{th}$, or $p_{ttest} > p_{th}$, it is confirmed that the copy number of the target gene of the sample to be tested is normal, wherein $CN_{thA}$, $CN_{thD}$, $sigRatio_{th}$, and $p_{th}$ are each independently thresholds.

In one aspect, the present application provides a copy number status analysis device, which can include modules for implementing the copy number status analysis method of the present application.

Databases, Devices, and Application Methods

In one aspect, the present application provides a database, which is established according to the copy number status analysis method or database establishment method in the present application.

In another aspect, the present application also provides a storage medium, which records programs capable of executing the methods in the present application.

In another aspect, the present application provides a device, which can include the storage medium in the present application. For example, the non-volatile computer-readable storage medium can include floppy disks, flexible disks, hard disks, solid-state storage (SSS) (such as solid-state drives (SSD)), solid-state cards (SSC), solid-state modules (SSM), enterprise flash drives, magnetic tapes, or any other non-transient magnetic media, etc. The non-volatile computer-readable storage medium can also include punch cards, paper tapes, optical discs (or any other physical medium with hole patterns or other optically recognizable marks), compact disc read-only memory (CD-ROM), rewritable CDs (CD-RW), digital versatile discs (DVD), Blu-ray discs (BD), and/or any other non-transient optical media.

For example, the device of the present application can also include a processor coupled to the storage medium, which can be configured to execute the methods in the present application based on the programs stored in the storage medium.

In another aspect, the present application also provides a method of the present application, which can be applied in disease diagnosis, prevention, and/or treatment.

In another aspect, the present application provides a method of the present application, which can be applied in monitoring the copy number status of target genes.

In another aspect, the present application provides a method of the present application, which can be applied in genome-wide association studies.

In the present application, the methods can be used to determine whether the subject has copy number variations. For example, one or more methods of the present application can be for non-diagnostic purposes. For example, one or more methods of the present application can be for diagnostic purposes.

In the present application, the methods can be used to detect copy number variations for clinical practice (for example, it can be inferred whether certain specific cancer treatments are suitable for the subject). In some cases, the level of copy number variations detected by the methods can be used in clinical practice in conjunction with biomarkers known in the field.

It is not intended to be limited by any theory, and the examples below are merely for illustrating the methods and applications of the present application, and not for limiting the scope of the invention of the present application.

## EXAMPLES

### Example 1

1.1 Data Preparation

Thirty negative peripheral blood samples were selected. Then, DNA was extracted from the peripheral blood using the same batch of experimental reagents, and a whole-genome pre-library was prepared through experimental steps such as fragmentation, adapter ligation, and PCR amplification. Next, the prepared pre-libraries were divided into two portions, which were hybridized with probes from different batches, referred to as batch A and batch B, to specifically capture the BRCA1 gene in the human genome, obtaining final libraries A and B. High-throughput sequencing was performed on both final libraries using a sequencer. Finally, the sequencing data were aligned with the human genome standard sequence hg19 to obtain the aligned BAM files.

1.2 Traditional Method for Detecting Copy Number Variations Based on Constructing Reference Baselines

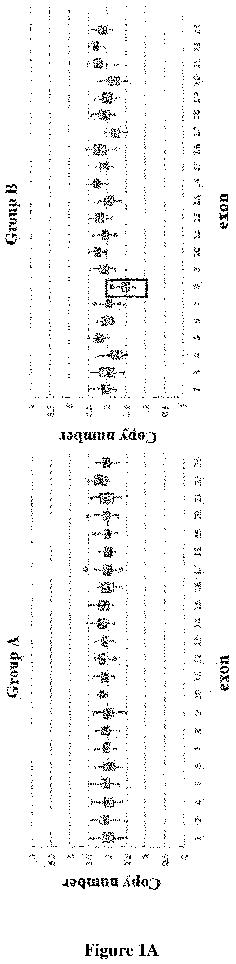

In advance, a sufficient number of negative samples with normal copy numbers (for example, 50 or more) collected earlier were used as a reference set to construct a reference baseline. Subsequently, the copy number values of each exon on the BRCA1 gene and the detection of copy number variations were calculated for the two groups of experimental samples using the baseline constructed from this reference set. From the calculated exon copy number results (as shown in Figure 1A), the experimental data captured by batch A probes were more uniform and closer to the theoretical copy number of 2, while the results captured by batch B probes were relatively worse, especially for exon 8 of the BRCA1 gene, where all samples showed a significant preference for lower values. In another aspect, from the results of copy number variation detection, 2 cases of false-positive copy number variations from BRCA1 were detected in the 30 samples tested with batch B probes. This indicated that the possible probe batch differences could easily lead to a decrease in the accuracy of copy number variation detection when using the traditional method based on reference baselines.

1.3 Detection of Copy Number Variations Based on the Method of the present application Therefore, copy number variations were detected using the method of the present application.

(1) Data Preparation

The copy number variation detection algorithm of the present application allowed for a selection of a sufficient number of samples, for example, 15 samples from the same sample type and experimental methodology, ensuring as much consistency as possible in the batches of reagents and experimental equipment used in the experiment. The data for each participating sample were required to come from BAM files aligned from NGS sequencing data.

(2) Deduplication Based on BAM Files and Statistical Analysis of DNA Sequence Fragment Coverage Depth

For BAM file of each sample, duplicate DNA sequence fragments introduced by PCR in the NGS library construction were first removed, obtaining unique aligned DNA fragments. Then, based on the target DNA region to be detected, a sliding window method was employed, sliding 24bp at a time, dividing the region into fixed-length probe windows of 120bp, and calculating the average coverage level of uniquely aligned DNA fragments in each window.

(3) Quality Control of Sample Sequencing Coverage

Optionally, quality control was performed on each sample to assess whether the average sequencing depth, minimum sequencing depth, and coverage uniformity met the specified requirements. Wherein, the requirements were an average sequencing depth $\geq$ 100X, minimum sequencing depth $\geq$ 30X, and coverage uniformity $\geq$ 90%. The coverage uniformity refers to the percentage of bases with a sequencing depth $\geq$20% of the sample average sequencing depth, and calculates as follows: Coverage Uniformity = (Number of bases $\geq$20% of the sample average sequencing depth / Total number of bases in the sample) $\times$ 100%). If the sample data quality did not meet the requirements, the data was excluded from the construction of the correction baseline. The detection method of the present application could test at least 10 samples that met the quality criteria.

(4) Data Correction and Normalization

To reduce the impact of noise and systematic bias on the detection results of copy number variations, the coverage level of each window region was corrected, including preliminary correction of the coverage level (based on the average coverage level of the sample), GC correction, and batch correction.

(5) Preliminary Correction of Coverage Level

To correct for differences in sequencing coverage depth between different samples, the preliminary coverage level correction adjusted the coverage level of all samples in the batch to the same specified coverage level. Specifically, for each window region in the batch samples, the obtained average coverage level was divided by the total of the average coverage levels of all window regions within the sample, then multiplied by a fixed factor (the factor was 1E+07).

(6) GC Correction

To correct for differences in sequencing coverage depth caused by GC bias, GC correction was performed by calculating the GC content of each window, and then using the loess regression method to correct for GC bias in the coverage level of each window region within the sample.

(7) Batch Correction

i. GC-corrected data for all quality-controlled samples within the batch were obtained.

ii. The median and median absolute deviation (MAD) of the coverage levels in each window for the samples involved in constructing the batch baseline were calculated. If MAD/median > a set threshold (for example, the threshold could be set to approximately 0.05 to approximately 0.15), it indicated that the coverage level of that window was unstable and needed to be excluded.

iii. Windows with MAD/median < the set threshold were retained, or the windows with the lowest 4 MAD/median values were kept as the regions with stable coverage levels.

iv. Subsequently, for each retained window region with a stable coverage level, Grubbs' test was used to remove outlier coverage level values within the window, and then the average level of the remaining coverage levels was calculated to serve as the batch correction reference coefficient.

v. Finally, for each sample to be tested, based on the calculated batch correction reference coefficient, the coverage level of each window region was normalized, and the copy number CN value was calculated, with the formula for calculating the CN value of each window as follows:

$$CN\ value = \frac{coverage\ level\ of\ the\ window\ area\ of\ the\ sample\ to\ be\ tested}{batch\ correction\ reference\ coefficient\ of\ the\ window\ area} * 2$$

(8) Identification of Copy Number Variations

The CBS algorithm was used to identify breakpoint positions on the sample target region, obtaining candidate copy number variation regions. Then, each candidate copy number variation region underwent a significance test. Specifically, through a T-test, it was determined whether the window coverage level of the sample to be tested in the candidate copy number variation region had a significant difference compared to the coverage level of other samples within the batch in that region, thereby assessing the reliability of the candidate copy number variation.

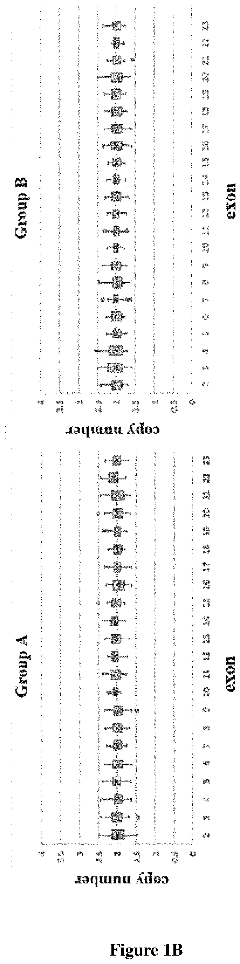

Wherein, as shown in Figure 1B, the distribution of BRCA1 gene exon copy numbers demonstrates that, compared to the traditional method based on constructing a reference baseline, the method of the present application achieved better uniformity of copy number results, especially for batch B probes with significant batch differences, with a more pronounced effect, and no false-positive copy number variations were detected in both groups of experimental data.

**Example 2**

Twenty cell line samples were selected, of which nineteen were negative samples, and one was a known exon copy number variation (large genomic rearrangement, LGR) sample (BRCA1: Exon 12 amp). The experiment utilized an automated library preparation method to obtain high-throughput sequencing data. Finally, the sequencing data were aligned with the human genome standard sequence hg19 to obtain aligned BAM files. The sample BAM files were analyzed for copy number variations using both the traditional method based on constructing a reference baseline and the method of the present application. The baseline used in the method based on constructing a reference baseline could be established using sample data from earlier manual library preparations (for example, the reference baseline used in Example 1).

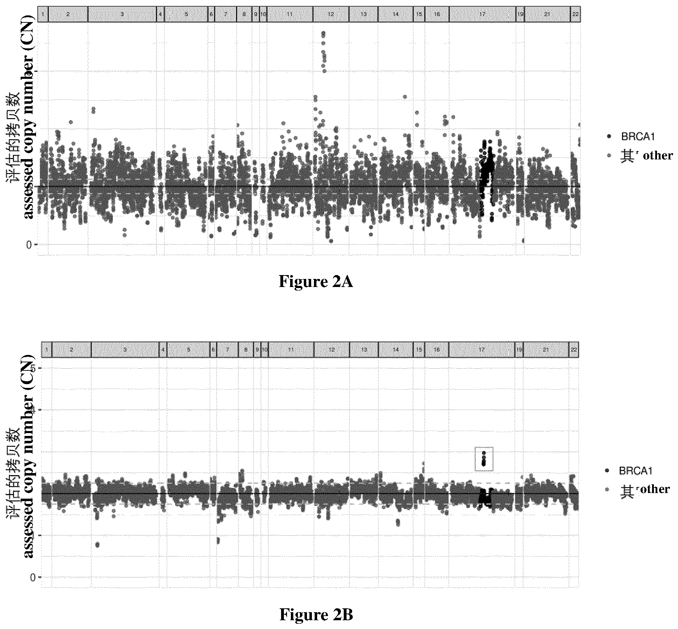

The results of the positive sample containing copy number variations, as shown in Figures 2A-2B, indicated that the traditional method based on constructing a reference baseline (shown in Figure 2A) exhibited significant background noise, making it impossible to detect copy number variations. In contrast, the method of the present application showed considerably less background noise, allowing for the detection of copy number variations (as shown in Figure 2B). This suggested that NGS data generated by different experimental methods may vary significantly, and baselines constructed from data obtained via manual library preparation methods were not suitable for data from automated library preparations. This indicated that when experimental methods change, if the traditional reference baseline approach was used, it was necessary to first collect a sufficient amount of sample data using the experimental method, and then manually construct a new baseline, significantly increasing the experimental costs and manpower waste.

**Example 3**

A total of 696 peripheral blood samples were selected for the detection of exon copy number variations (LGR) in BRCA1 and BRCA2. The experiment used RNA probes to specifically capture the gene regions of BRCA1 and BRCA2, followed by high-throughput sequencing. The sequencing data were then aligned with the human genome standard sequence hg19 to obtain aligned BAM files. Subsequently, copy number variations were detected using both the method based on constructing a reference baseline and the method of the present application. In addition, all sample copy number variations were confirmed using the BRCA MASTR Plus Dx kit (based on multiplex PCR capture methodology), including a total of 17 LGR positive samples and 679 negative samples.

Taking the detection results of the BRCA MASTR Plus Dx kit as a benchmark, the sensitivity and specificity of the detection results for the 696 peripheral blood samples using the traditional method based on constructing a reference baseline and the method of the present application were obtained, as shown in Table 1 and Table 2 respectively.

Table 1. Detection Results Based on the Method of Constructing a Reference Baseline

|  |  | detection results based on MASTR |  | Detection Index |
|---|---|---|---|---|
|  |  | positive | negative |  |
| detection results based on reference baseline | positive | 12 | 167 | positive Predictive Value 67% |
|  | negative | 5 | 512 | negative Predictive Value 99% |
| Detection Index |  | Sensitivity 70.6% | Specificity 75.4% | Accuracy 75.3% |

Table 2. Detection Results Based on the Method of the Present Application

|  |  | detection results based on MASTR |  | Detection Index |
|---|---|---|---|---|
|  |  | positive | negative |  |
| detection results based on the present application | positive | 12 | 3 | positive Predictive Value 80% |
|  | negative | 5 | 676 | negative Predictive Value 99.3% |
| Detection Index |  | Sensitivity 70.6% | Specificity 99.6% | Accuracy 98.9% |

Comparing Table 1 and Table 2, it could be observed that, compared to the traditional method of constructing a baseline, the method of the present application could significantly reduce false positives without losing sensitivity, improving detection accuracy from 75.3% to 98.9%.

**Example 4**

Data from 14 cell line samples were selected for sequencing and alignment to construct batch baselines. During the construction of the batch baselines, thresholds for describing window coverage fluctuation levels were set at 0.05 and 0.15, respectively, to construct two batch baselines. The two batch baselines were then used to batch-correct the samples known to have LGR copy number variations (BRCA1: exon4-6del) among the 14 samples, before detecting copy number variations.

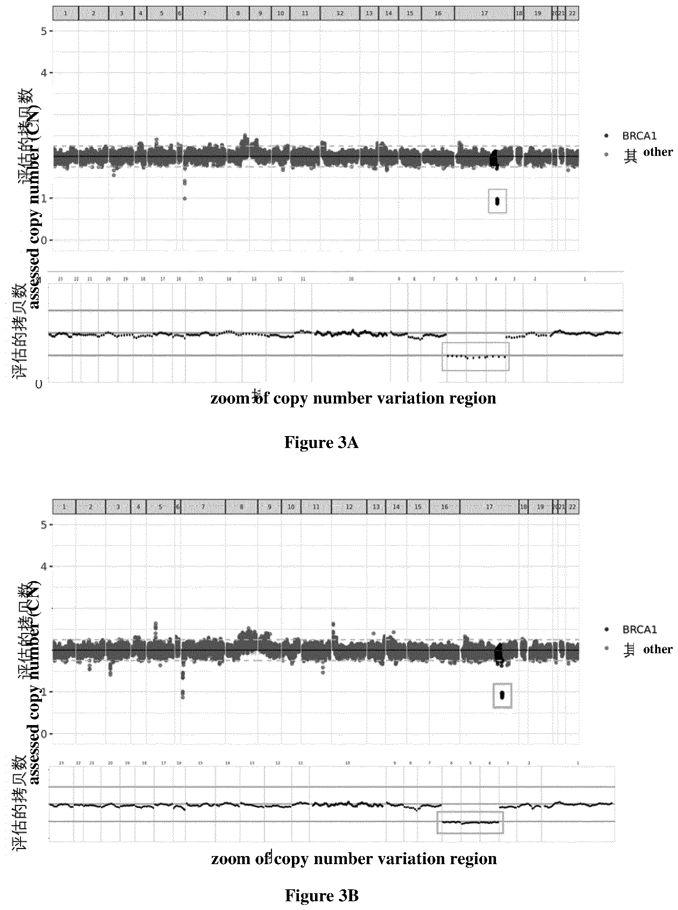

The results of the positive samples containing copy number variations, as shown in Figures 3A-3B, indicated that batch baselines constructed based on different window coverage fluctuation level thresholds could both clearly detect copy number variations, demonstrated that the threshold range selected for stable regions in the present application could achieve the detection of copy number variations.

**Example 5**

10 negative cell line samples were selected as simulated sample backgrounds, and then 10 LGR copy number variations in the BRCA1 and BRCA2 genes, which had been reported in the literature, were chosen as the mutations to be simulated (as shown in Table 3), including five types of copy number amplification variations and five types of copy number deletion variations. Through simulation, the aforementioned copy number amplification variations and copy number deletion variations were artificially introduced into the simulated sample background data, resulting in 10 positive LGR simulated sample data.

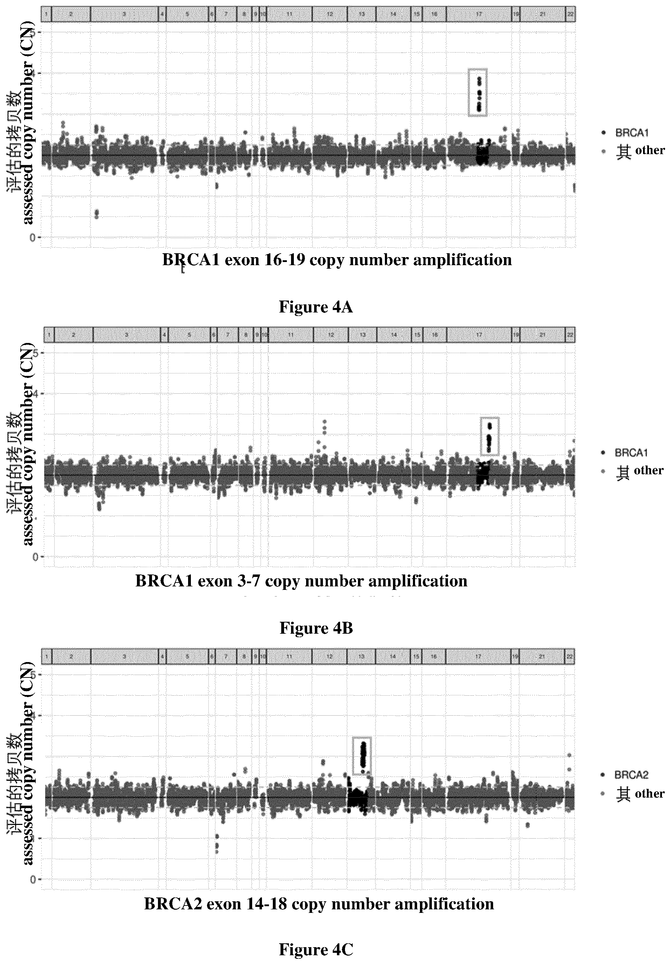

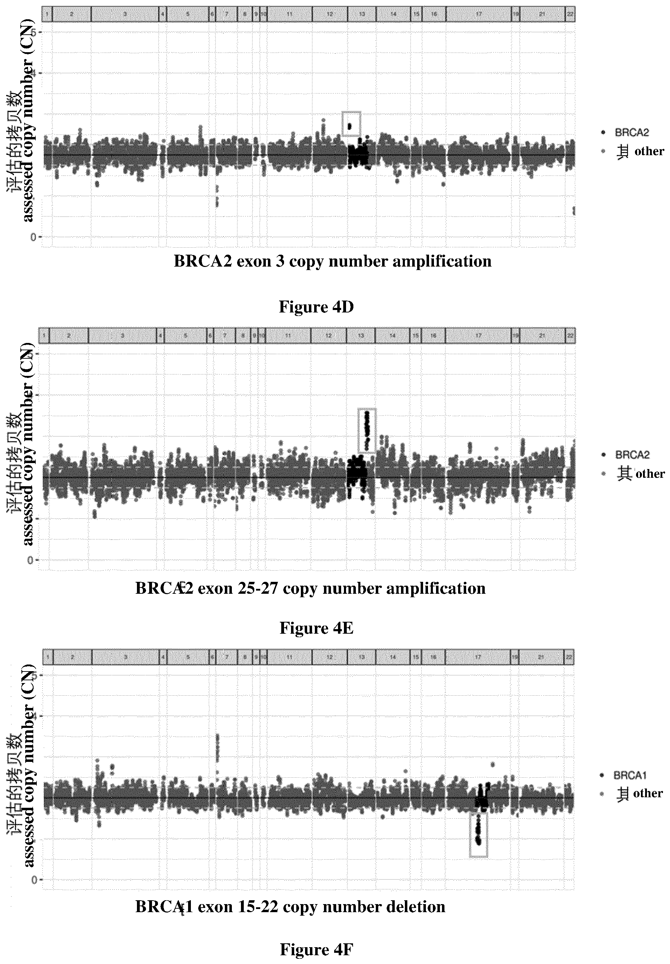

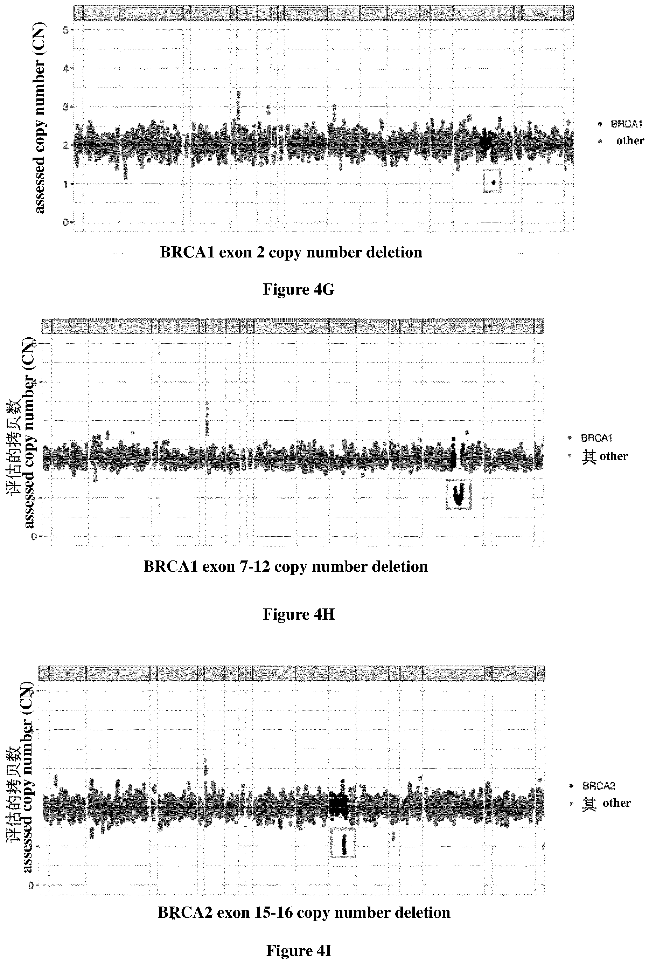

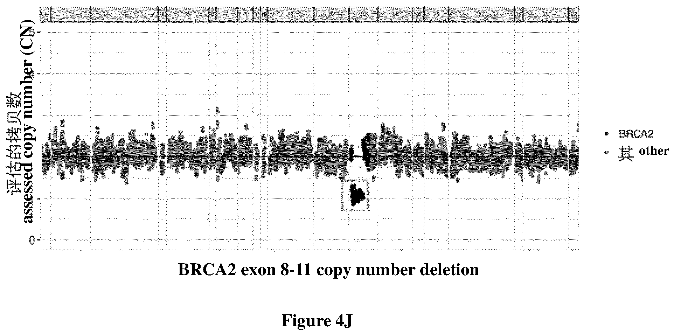

A batch baseline was constructed using the 10 simulated positive samples, and then the constructed batch baseline was used for batch correction and identification of copy number variations in the 10 simulated samples. The results of the 10 simulated samples, as shown in Figures 4A-4J, indicated that all 10 simulated copy number variations could be accurately detected, demonstrating that the present application could achieve accurate detection of copy number variations in any region.

Table 3: Types of Copy Number Variations in the 10 Simulated Samples

| Name of Figure | gene | copy number variation |
|---|---|---|
| BRCA1 exon 16-19 copy number amplification (Figure 4A) | *BRCA1* | exon 16-19_Dup |
| BRCA1 exon 3-7 copy number amplification (Figure 4B) | *BRCA1* | exon 3-7_Dup |
| BRCA2 exon 14-18 copy number amplification (Figure 4C) | *BRCA2* | exon 14-18_Dup |
| BRCA2 exon 3 copy number amplification (Figure 4D) | *BRCA2* | exon 3_Dup |
| BRCA2 exon 25-27 copy number amplification (Figure 4E) | *BRCA2* | exon 25-27_Dup |
| BRCA1 exon 15-22 copy number deletion | *BRCA1* | exon 15-22_Del |
| (Figure 4F) | | |
| BRCA1 exon 2 copy number deletion (Figure 4G) | *BRCA1* | exon 2_Del |
| BRCA1 exon 7-12 copy number deletion (Figure 4H) | *BRCA1* | exon 7-12_Del |
| BRCA2 exon 15-16 copy number deletion (Figure 4I) | *BRCA2* | exon 15-16_Del |
| BRCA2 exon 8-11 copy number deletion (Figure 4J) | *BRCA2* | exon 8-11_Del |

**Example 6**

The method of the present application, using cluster method, divided a large number of real sample data into different sample sets based on trends in sequencing depth clustering, and constructed baselines (average depth and depth fluctuation range) for each. Based on the similarity between the sample and the baseline, background baselines were dynamically selected to eliminate batch effects while improving the specificity and sensitivity of detection. Additionally, discrete wavelet transform methods could optionally be used for copy number smoothing and denoising, enhancing the signal-to-noise ratio of sequencing data.

To provide a method and medium for detecting copy number variations in high-throughput sequencing with high sensitivity and accuracy , the method of the present application relied on detecting copy number variations based on the specificity of sample coverage characteristics. Specifically, through cluster analysis of large-scale samples and the construction of multiple control group baselines, the method could avoid baseline mismatch issues caused by inconsistencies in coverage depth characteristics due to experimental and sample differences. Moreover, the method could integrate various coverage depth correction strategies to reduce data differences specific to samples. Ultimately, through quantitative analysis and statistical difference assessment, the accuracy and stability of the results were ensured. The method for detecting copy number variations in the present application could be applied not only to targeted capture sequencing data of specific gene panels but also to whole-exome capture sequencing data. To address the above issues, the database establishment method of the present application could include the following steps:

1. Data Preparation Module, which included:

a) Sequence Alignment: High-throughput sequencing raw fastq data were aligned to the human reference genome, determining sequences matching the human reference genome reference sequence in the target regions of the sample to be tested and reference samples.

b) Removal of Duplicate Sequences: Duplicate sequences generated during the PCR amplification process were removed.

c) Coverage Depth Calculation: The sequencing depth $RD_{Base}$ for each base in the target region was calculated.

2. Coverage Depth Correction Module, encompassing three independent, sequentially optional corrections:

a) Normalization of Sequencing Data: Total sequencing depth correction for samples was conducted, specifically, by normalizing the coverage depth at each site of the target regions according to the total sequencing depth of the samples, to eliminate differences in sequencing data volume between different samples, obtaining $RD_{normD}$:

$$RD_{normD} = \frac{RD_i}{\sum_{i=1}^{n} RD_i} * R(R = 1e + 07)$$

wherein, i represents the site on the target region, n represents the total number of sites on all target regions, $RD_i$ represents the sequencing depth at site i on the target region, and R is a constant that can be set based on the size of all regions, to ensure that the depth of the sample to be tested after correction is on the same level as that of the reference sample group after correction.

b) Smoothing of Sequencing Data 1: Probe layout feature correction could specifically involve correcting the sequencing depth on target regions based on differences in probe layout multipliers, such as the number of probes covering a region. The regions were divided, each target region length could be approximately 24 base pairs, and the average coverage depth RD for each target region was calculated. Based on the number of probes covering each target region ProbeN, local weighted regression (loess(RD-ProbeN)) correction of the sequencing depth on the target regions was performed, obtaining the probe-corrected sequencing depth $RD_{normP}$.

c) Smoothing of Sequencing Data 2: GC Correction involved extending the target regions used for coverage depth calculation by flanks to a total length greater than 200bp, calculating the average GC ratio, and performing local weighted regression (loess(RD-GC)) correction on the sequencing depth RD based on the GC content GC of each region, obtaining the GC-corrected sequencing depth $RD_{normGC}$.

d) Optionally, regions with balanced GC content could be selected, filtering out regions with extreme GC imbalance (regions with a CG content below 0.25, or GC content above 0.75), using the corrected coverage depth for copy number variation detection.

3. The baseline construction module specifically included the following steps:

a) Sample clustering: Traditional methods usually consider all reference samples as a single category for baseline construction. The method in the present application grouped the reference samples, specifically, based on the consistency of the coverage depth of each reference sample over the target region, such as the degree of approximation of the sequencing of the reference samples on the target region. Cluster analysis was performed, and the reference samples were divided into different categories of reference sample groups. The clustering methods could be, for example, K-means clustering, hierarchical clustering, etc.

b) Baseline Construction: For each reference sample group, a baseline was constructed separately. Specifically, the average sequencing depth ( $MeanRD_{Baseline}^{i}$ ) and the standard deviation of sequencing depth ( $SdRD_{Baseline}^{i}$ ), across all target regions for each reference sample group were calculated, serving as the baseline ($Baseline_i$), wherein i={ 1,2,3,4,...}. For example, each reference sample group needed to have a sufficient number of samples to be statistically significant, with no fewer than 30 samples per reference sample group. The number of reference sample groups to be set took into consideration the characteristics of tumor samples and sequencing quality, determined by the number of captured features, for example, the number of reference sample groups could be more than two, such as 2-10.

c) Optionally, region selection could be performed: The coefficient of variation (cv) of sequencing depth on each region was calculated, and regions with high coverage fluctuation levels in the samples, indicating instability, were eliminated, wherein:

$$cv^i = \frac{SdRD^i_{Baseline}}{MeanRD^i_{Baseline}}$$

wherein, $MeanRD^i_{Baseline}$ and $SdRD^i_{Baseline}$ represent the average sequencing depth and the standard deviation of sequencing depth across all target regions, respectively. When cv>0.8, the region was considered unstable and filtered out; likewise, corrected sequencing depths lower than 5 were considered regions of low capture efficiency and were filtered out, leaving the remaining regions as stable regions.

Ultimately, according to the Example, the database in the present application, which included two or more reference sample groups with consistency in variation across target regions, was obtained. Compared to existing technologies, the advantage of the database in the present application was: through large-scale sample cluster analysis, reference samples were divided into different categories of reference sample groups, constructing sample-specific background baselines, significantly reducing the false positives produced by batch effects in the detection of copy number variations in high-throughput sequencing data, and increasing the stability of results. Additionally, the method in the present application to eliminate batch effects did not require ensuring a sufficient number of samples of the same gene panel within the same batch, greatly reducing the difficulties in practical applications.

**Example 7**

To address the issue of baseline mismatch caused by inconsistencies in coverage depth features due to experimental and sample variations in sequencing, the present application also provided a copy number status analysis method. The copy number status analysis method in the present application could include the following steps:

a) based on the similarity between the sample to be tested and the reference sample group, the reference sample group closest to the sample to be tested was determined, that is, the baseline was dynamically selected: By using statistical distance calculation methods, such as the Minkowski distance, the sequencing depth of the sample to be tested on each target region was compared with the sequencing depth of each reference sample group on that target region, confirming the statistical distance between the reference sample group and the sample to be tested:

$$L_p(RD_{Sample}, RD_{Baseline}) = \left(\sum_{i=1}^{n} |RD^i_{Sample} - RD^i_{Baseline}|^p\right)^{\frac{1}{p}}$$

wherein, the $L_p$ value represents the statistical distance, i represents the target region, n represents the number of target regions, $RD^i_{Sample}$ represents the sequencing depth of the target region i of the sample to be tested, $RD^i_{Baseline}$ represents the sequencing depth of the target region i of the reference sample group, wherein p is optionally greater than or equal to 1. The reference sample group with the smallest statistical distance (the smallest $L_p$ value indicating the highest similarity) to the sample to be tested was selected as the background baseline ($Baseline_x$) for the sample to be tested.

b) Gene copy number detection, specifically as follows:

i. Copy number assessment of target regions for each sample to be tested: The copy number $CN_i$ for each target region of the sample to be tested was calculated, with the formula as follows:

$$CN_i = 2 * \frac{RD_{sample}}{RD^x_{Baseline}}$$

wherein, $RD_{sample}$ represents the sequencing depth of each target region of the sample to be tested,

$RD^x_{Baseline}$ represents the sequencing depth of each target region of the reference sample group closest to the sample to be tested, with the ploidy being potentially 2.

ii. Optionally, smoothing and denoising for the copy number of each region could be performed: Denoising algorithms were used to smooth the $CN_i$ of each region, improving the signal-to-noise ratio of the data. Denoising methods could employ Discrete Wavelet Transformation (DWT), Principal Component Analysis, Singular Value Decomposition, and/or Gaussian filtering techniques for smoothing and denoising. DWT involves dividing the signal into high and low frequency signals, passing them through low-pass and high-pass filters respectively, performing discrete wavelet transformation on the discrete signals, and discretizing the continuous wavelets and their transformations to achieve the purpose of data denoising. Thus, the noise-reduced $CN_i$ was obtained;

iii. The copy number assessment for each target gene: the weighted average copy number $CN_g$ of each target gene in the sample was calculated, and the $CN_i$ was corrected using the length of the target exon, for example:

$$CN_g = \frac{\sum_j^m(\frac{\sum_i^n(CN_i)}{n} * Len_j)}{\sum_j^m Len_j}$$

wherein i represents the target region, j represents the target exon, n represents the number of target regions on target exon j, m represents the number of target exons, $CN_i$ represents the copy number of target region i, $Len_j$ represents the length of target exon j.

iv. The probability of the presence of copy number variations in the sample to be tested on the target regions was determined, for example, by performing normality tests on the sequencing depth of multiple regions and the distribution of the selected background baseline, with the formula as follows:

$$p_a = pnorm(RD^i_{Sample}, mean = MeanRD^x_{Baseline}, sd = SdRD^x_{Baseline}, lower. tail = F)$$

$$p_d = pnorm(RD^i_{Sample}, mean = MeanRD^x_{Baseline}, sd = SdRD^x_{Baseline}, lower. tail = T)$$

wherein, $RD^i_{Sample}$ represents the sequencing depth of target region i of the sample to be tested, $MeanRD^x_{Baseline}$ represents the mean sequencing depth of each target region of the reference sample group closest to the sample to be tested, $SdRD^x_{Baseline}$ represents the standard deviation of the sequencing depth of each target region of the reference sample group closest to the sample to be tested. The right tail probability is calculated with *lower. tail = F*, $p_a$ represents probability value of copy number amplification occurring in that region, The left tail probability is calculated with *lower. tail = T*, $p_d$ represents probability value of copy number deletion occurring in that region.

v. The significant proportion assessment for each target gene: the proportion of significant amplification or deletion, sigRatio, for each target gene separately, was calculated, with the formula as follows:

$$sigRatio = \frac{\text{Number of target regions where significant copy number variations occur}}{\text{Number of all target regions on the target gene}}$$

wherein, the target regions where significant copy number variations occur include the target regions with a proportion of copy number variations of approximately 30% or higher.

vi. Comprehensive level significance test for each target gene: A T-test was conducted on the corrected sequencing depth of each target region on each target gene based on the average sequencing depth of the regions of the reference sample group closest to the sample to be tested, to determine whether the difference between the sample and the baseline was significant, resulting in $p_{ttest}$.

c) The copy number status of the target gene of the sample to be tested was determined, with the judgment criteria:

$$\text{copy number status}$$

$$= \begin{cases} amplification\colon\ CN_g \geq CN_{thA}\ AND\ sigRatio \geq sigRatio_{th}\ AND\ p_{ttest} \leq p_{th} \\ deletion\colon\ CN_g \leq CN_{thD}\ AND\ sigRatio \geq sigRatio_{th}\ AND\ p_{ttest} \leq p_{th} \\ normal\colon\ CN_{thD} < CN_g < CN_{thD}\ OR\ sigRatio <\ sigRatio_{th}\ OR\ p_{ttest} > p_{th} \end{cases}$$

Each threshold could be obtained through training with a large dataset. Wherein: $CN_{thA}$ represents the threshold for copy number amplification, with values that can optionally range from 2.25 to 4; $CN_{thD}$ represents the threshold for copy number deletion, with values that can optionally range from 1.0 to 1.75; $sigRatio_{th}$ represents the threshold for the proportion of significant amplification/deletion, with values that can optionally range from 0.3 to 1; $p_{th}$ represents the threshold for the significance in T-test, with values that can optionally range from 0.05 to 0.00001.

The copy number status analysis method in the present application dynamically selected the reference sample group closest to the sample to be tested as the background baseline based on the similarity between the sample to be tested and the reference sample group, which could eliminate batch effects and also improve the specificity and sensitivity of detection.

**Example 8**

Database Establishment: A baseline was constructed using 655 reference samples, employing the database establishment method in the present application, such as the k-means clustering algorithm, to divide the reference samples into 5 reference sample groups, thus constructing 5 different candidate baselines for the database.

Construction of Simulated Data: The VarBen tumor mutation data simulation software (github.com/nccl-jmli/VarBen) was used, based on benign tissue samples, to insert target gene reads into sequencing data through the insertion of gene reads, simulating different copy number amplifications of the target gene in a gradient manner. The list of simulated samples is shown in Table 4.

Table 4: List of Simulated Samples

| Target gene | Number of simulated samples | Simulate copy number gradient |
|---|---|---|
| ALK | 20 | 2.5, 2.75, 3.0, 3.5, 4.0 |
| ERBB2 | 20 | 2.5, 2.75, 3.0, 3.5, 4.0 |
| FGFR1 | 20 | 2.5, 2.75, 3.0, 3.5, 4.0 |
| FGFR2 | 20 | 2.5, 2.75, 3.0, 3.5, 4.0 |

The simulated samples were tested using the copy number status analysis method in the present application, and the detection results were shown in Table 5.

Table 5: Detection Results in Simulated Samples of the Present Application

| Target gene | Number of simulated copy number | Proportion of amplification detected of the present application | Averaged value of copy number detected of the present application | Copy number in the 95% confidence region of the detection of the present application |
|---|---|---|---|---|
| ALK | 2.5 | 100% | 2.58 | (2.41, 2.76) |
| | 2.75 | 100% | 2.84 | (2.64, 3.04) |
| | 3.0 | 100% | 3.11 | (2.94, 3.28) |
| | 3.5 | 100% | 3.41 | (3.19, 2.63) |
| | 4.0 | 100% | 3.96 | (3.69,4.22) |

(continued)

| Target gene | Number of simulated copy number | Proportion of amplification detected of the present application | Averaged value of copy number detected of the present application | Copy number in the 95% confidence region of the detection of the present application |
|---|---|---|---|---|
| ERBB2 | 2.5 | 100% | 2.58 | (2.36, 2.81) |
| | 2.75 | 100% | 2.8 | (2.6,3.18) |
| | 3.0 | 100% | 3.18 | (2.89,3.47) |
| | 3.5 | 100% | 3.50 | (3.14, 3.86) |
| | 4.0 | 100% | 4.08 | (3.68, 4.48) |
| FGFR1 | 2.5 | 100% | 2.55 | (2.40, 2.70) |
| | 2.75 | 100% | 2.84 | (2.66, 3.02) |
| | 3.0 | 100% | 3.12 | (2.94, 3.30) |
| | 3.5 | 100% | 3.40 | (3.21, 3.58) |
| | 4.0 | 100% | 3.98 | (3.78, 4.19) |
| FGFR2 | 2.5 | 100% | 2.6 | (2.38, 2.82) |
| | 2.75 | 100% | 2.92 | (2.69, 3.15) |
| | 3.0 | 100% | 3.25 | (2.97, 3.53) |
| | 3.5 | 100% | 3.57 | (3.33, 3.82) |
| | 4.0 | 100% | 4.17 | (3.89, 4.45) |

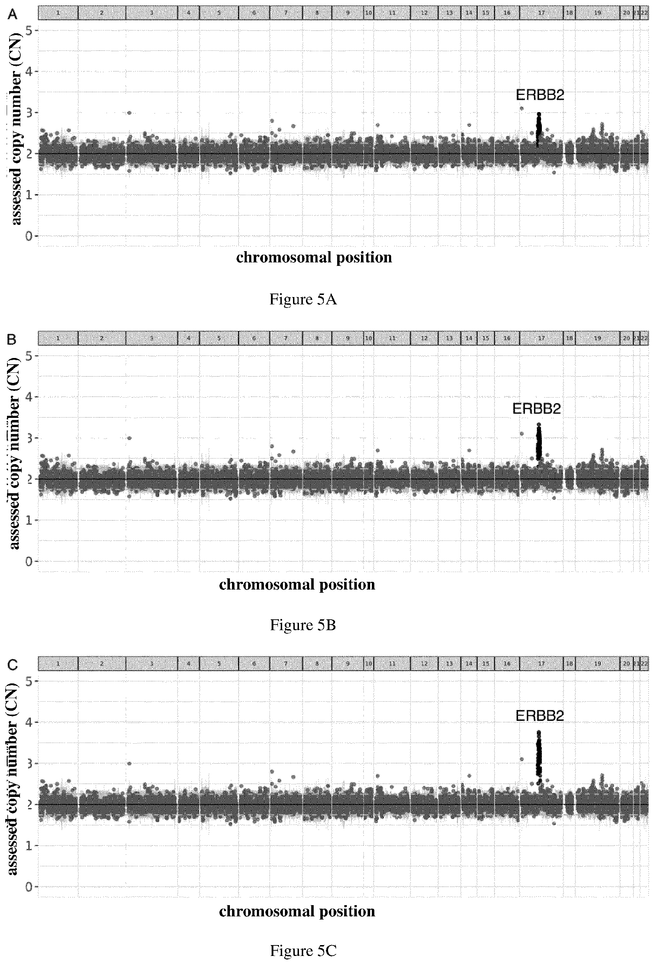

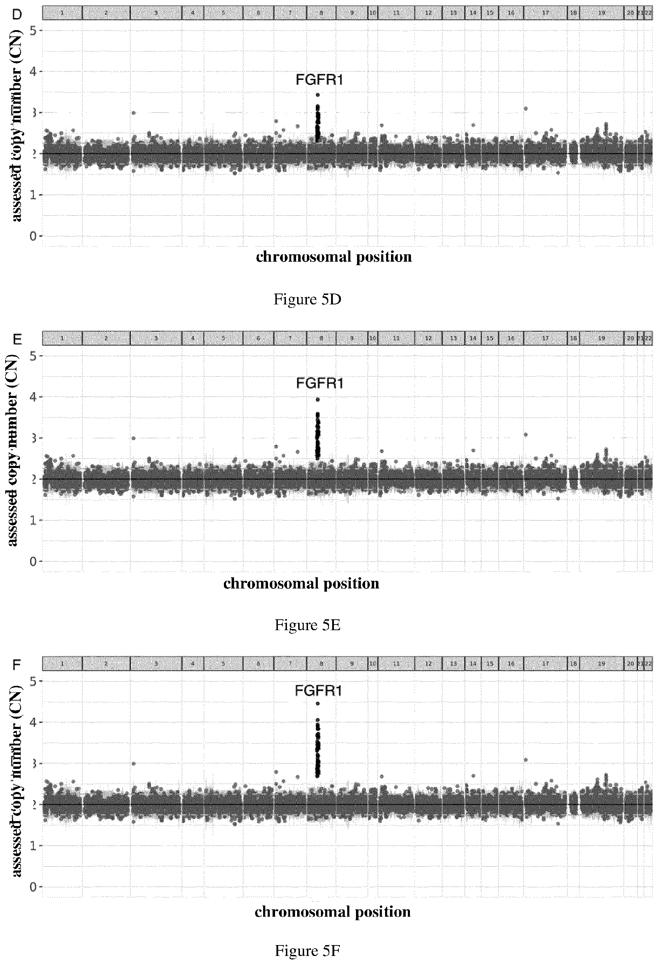

Figures 5A-5F showed examples of copy number distribution data from the detection results of the present application. Each point represented a region of a gene, with gray points indicating genes with normal copy numbers and black points indicating genes with amplifications or deletions, also marking the corresponding gene names. The horizontal axis represented the chromosomal position of the genes, while the vertical axis represented the copy numbers calculated using the method of the present application (the middle horizontal line indicated the copy number of normal genes), with the gray background indicating the range of fluctuations in each target region within the background baseline (the reference sample group closest to the sample to be tested). Figures 5A-5C represented different degrees of copy number amplification simulated for the ERBB2 gene, and Figures 5D-5F represented different levels of copy number amplification for the FGFR1 gene, with simulated copy number gradients of 2.5, 2.75, and 3.0. The results indicated that the copy number status analysis method of the present application, when used in simulated samples, could stably detect all simulated genes and different gradients of copy number amplification, with accurate copy number prediction.

**Example 9**

Positive Standard Samples: The tests conducted in the present application included 30 positive standard samples from the NCI-BL2009 cell line, where the target genes were transfected into the cell line using plasmid transfection to obtain CNV positive data, and the copy numbers of the genes were quantified using droplet digital PCR (ddPCR). The plasmid numbers were: Life RPCI11.C-433C10 BAC-EGFR, Life RPCI11.C-936I7 BAC-CDK4, Life RPCI11.C-163C9 BAC-MET, Life RPCI11.C-909L6 BAC-ERBB2, and Life RPCI11.C-957P17 BAC-FGFR1. The list of positive standard samples was shown in Table 6.

Table 6: List of Positive Standard Samples

| gene | Number of standard samples | ddPCR calibrated copy number |
|---|---|---|
| CDK4 | 10 | 3, 5, 8 |
| ERBB2 | 10 | 3, 5, 8 |
| EGFR | 10 | 3, 5, 8 |

(continued)

| gene | Number of standard samples | ddPCR calibrated copy number |
|------|------|------|
| FGFR1 | 10 | 3, 5, 8 |
| MET | 10 | 3, 5, 8 |

Database Establishment: A baseline was constructed using 655 reference samples, employing the database establishment method of the present application, such as the k-means clustering algorithm, to divide the reference samples into 5 reference sample groups, thus constructing 5 different candidate baselines for the database.

The copy number status of copy number amplification positive standard samples was tested using the copy number status analysis method of the present application, and the detection results were shown in Table 7.

Table 7: Detection Results in Positive Standard Samples by the Present Application

| gene | ddPCR calibrated copy number | Proportion of amplification detected of the present application | Averaged value of copy number detected of the present application | Copy number in the 95% confidence region of the detection of the present application |
|------|------|------|------|------|
| CDK4 | 3 | 100% | 2.84 | (2.68, 3.00) |
| | 5 | 100% | 4.86 | (4.70, 5.02) |
| | 8 | 100% | 7.60 | (7.30, 7.90) |
| ERBB2 | 3 | 100% | 2.70 | (2.70, 2.70) |
| | 5 | 100% | 4.17 | (4.03, 4.32) |
| | 8 | 100% | 6.51 | (6.24, 6.78) |
| EGFR | 3 | 100% | 2.70 | (2.70, 2.70) |
| | 5 | 100% | 4.43 | (4.28, 4.57) |
| | 8 | 100% | 7.03 | (6.80, 7.26) |
| FGFR1 | 3 | 100% | 2.90 | (2.90, 2.90) |
| | 5 | 100% | 4.77 | (4.63, 4.92) |
| | 8 | 100% | 7.53 | (7.24, 7.81) |
| MET | 3 | 100% | 2.91 | (2.80, 3.03) |
| | 5 | 100% | 4.66 | (4.50, 4.82) |
| | 8 | 100% | 7.26 | (7.02, 7.49) |

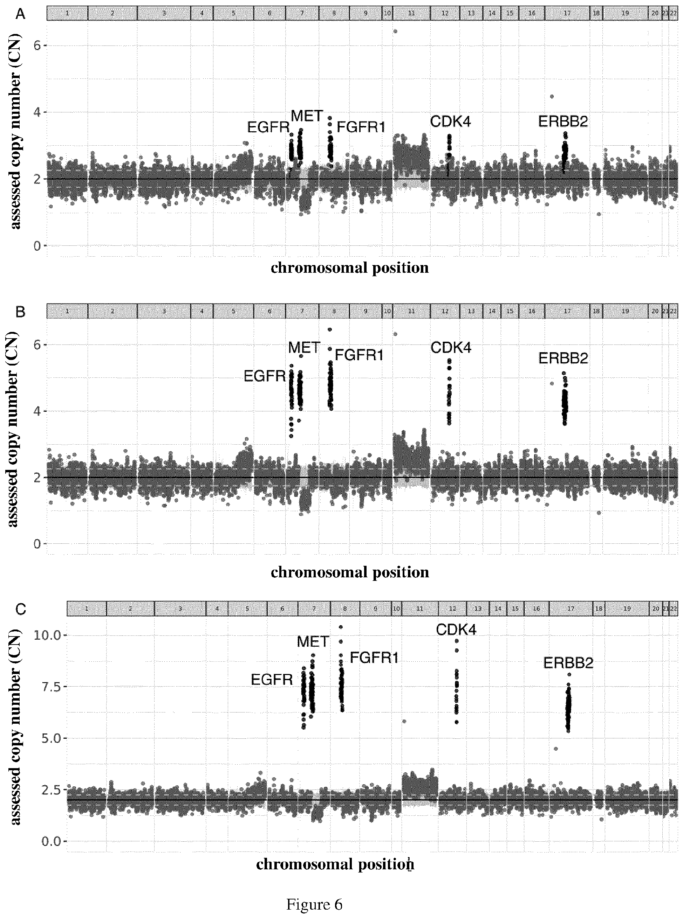

Figures 6A-6C showed examples of copy number distribution data from the detection results of the present application. Figures 6A-6C represented the detection results of CNV positive cell line standard samples transfected with plasmids, with ddPCR calibrated copy numbers of 3, 5, and 8 respectively. The results indicated that the method of this invention, when applied to plasmid-transfected cell line standards, could stably detect all genes and different copy number states, with accurate copy number prediction.

**Example 10**

Real Data: The real samples tested in the present application included 20 ERBB2 amplification positive samples verified by third-party immunohistochemistry (IHC). The list of real samples were shown in Table 8.

Table 8: List of Real Samples

| gene | Number of samples | IHC result |
|------|------|------|
| ERBB2 | 20 | Copy number: 3+ |

Database Establishment: A baseline was constructed using 443 reference samples, employing the database establishment method of the present application, such as the k-means clustering algorithm, to divide the reference samples into reference sample groups, thus constructing different candidate baselines for the database.

The copy number status of real samples was tested using the copy number status analysis method of the present application, and the results were shown in Table 9.

Table 9: Detection Results in Real Samples of the Present Application

| gene | Proportion of amplification detected of the present application | Range of copy number detected of the present application | Averaged value of copy number detected of the present application |
|---|---|---|---|
| ERBB2 | 100% | 3.27~6.66 | 4.7485 |

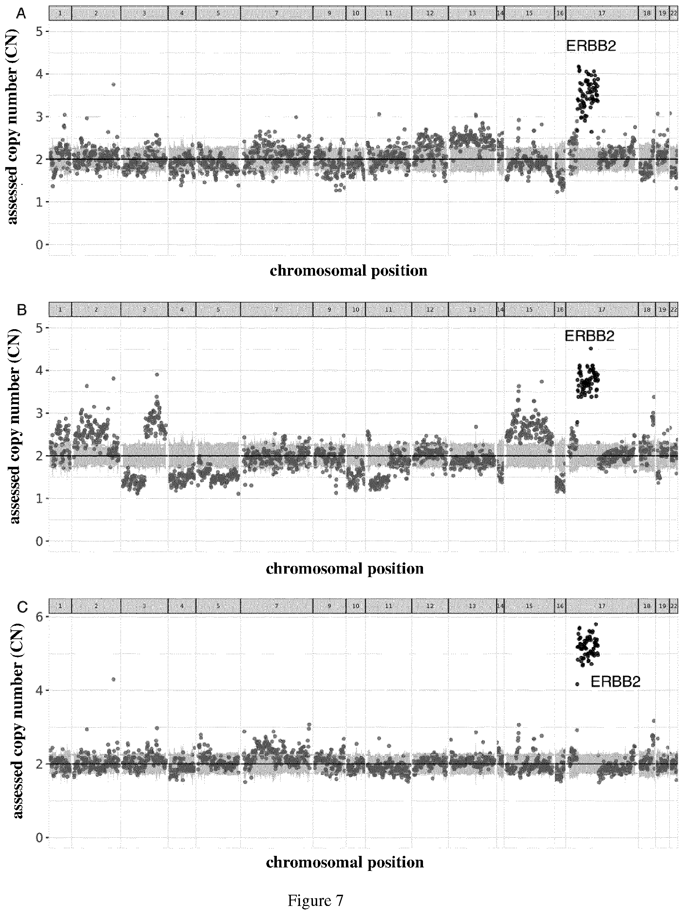

Figures 7A-7C showed examples of copy number distribution data from the detection results of the present application. Figures 7A-7C represented the detection results of real ERBB2 positive samples. The results indicated that the method of the present application, when applied to real samples, could stably detect all 20 samples with HER2 positive results from

IHC.

**Example 11**

Positive Standard Samples: The present application included 3 positive standard samples, sourced as in Example 9, for detecting the results with different baselines. The list of positive standard samples were shown in Table 10.

Table 10: List of Positive Standard Samples

| Samples | gene | ddPCR calibrated copy number |
|---|---|---|
| Standard Sample 1 | CDK4, ERBB2, EGFR, FGFR1, MET | 3 |
| Standard Sample 2 | CDK4, ERBB2, EGFR, FGFR1, MET | 5 |
| Standard Sample 3 | CDK4, ERBB2, EGFR, FGFR1, MET | 8 |

Database Establishment: A baseline was constructed using 655 reference samples, utilizing the database establishment method of the present application, such as the k-means clustering algorithm, to divide the reference samples into 5 reference sample groups, thus creating 5 different candidate baselines for the database. Additionally, without employing the cluster method, all reference samples were used to construct 1 baseline.

The 5 baselines obtained from the clustering algorithm and the 1 baseline obtained without clustering were used as references for detecting the copy number status of the standard samples with copy number amplification. The choice of baselines and the fluctuation in samples were shown in Table 11, with the results shown in Table 12.

Table 11: Fluctuation in Detection Results with Different Baselines

| samples | baseline | distance value between the sample and the baseline | SD of overall copy number of samples |
|---|---|---|---|
| standard sample 1 | baseline 1 (method of the present application) | 34.655 | 0.2378 |
| | baseline 2 | 66.018 | 0.4711 |
| | baseline 3 | 43.609 | 0.3223 |
| | baseline 4 | 60.49 | 0.5293 |
| | baseline 5 | 48.74 | 0.3685 |
| | 1 baseline constructed using all reference samples | 57.993 | 0.5107 |

(continued)

| samples | baseline | distance value between the sample and the baseline | SD of overall copy number of samples |
|---|---|---|---|
| standard sample 2 | baseline 1 (method of the present application) | 35.198 | 0.2356 |
| | baseline 2 | 66.625 | 0.4628 |
| | baseline 3 | 44.022 | 0.3172 |
| | baseline 4 | 61.076 | 0.5260 |
| | baseline 5 | 49.181 | 0.3573 |
| | 1 baseline constructed using all reference samples | 58.544 | 0.5095 |
| standard sample 3 | baseline 1 (method of the present application) | 34.94 | 0.2378 |
| | baseline 2 | 65.714 | 0.4670 |
| | baseline 3 | 44.089 | 0.3150 |
| | baseline 4 | 60.32 | 0.5297 |
| | baseline 5 | 48.457 | 0.3597 |
| | 1 baseline constructed using all reference samples | 57.749 | 0.5044 |

Table 12: Detection Results with Different Baselines

| gene | baseline | standard sample 1 | | standard sample 2 | | standard sample 3 | |
|---|---|---|---|---|---|---|---|
| | | copy number detected | detection result | copy number detected | detection result | copy number detected | detection result |
| CDK4 | baseline 1 (method of the present application) | 2.8 | amplification | 4.8 | amplification | 7.7 | amplification |
| | baseline 2 | 2.5 | normal | 4.2 | amplification | 6.7 | amplification |
| | baseline 3 | 2.8 | normal | 4.6 | amplification | 7.2 | amplification |
| | baseline 4 | 2.9 | normal | 4.8 | amplification | 7.6 | amplification |
| | baseline 5 | 2.7 | normal | 4.5 | amplification | 7.2 | amplification |
| | 1 baseline constructed using all reference samples | 3.1 | normal | 5.2 | amplification | 8.3 | amplification |
| ERBB2 | baseline 1 (method of the present application) | 2.7 | amplification | 4.2 | amplification | 6.4 | amplification |

(continued)

| gene | baseline | standard sample 1 | | standard sample 2 | | standard sample 3 | |
|---|---|---|---|---|---|---|---|
| | | copy number detected | detection result | copy number detected | detection result | copy number detected | detection result |
| | baseline 2 | 2.3 | normal | 3.7 | amplification | 5.6 | amplification |
| | baseline 3 | 2.5 | normal | 4 | amplification | 6.1 | amplification |
| | baseline 4 | 2 | normal | 3.2 | amplification | 4.9 | amplification |
| | baseline 5 | 2.4 | normal | 3.9 | amplification | 5.9 | amplification |
| | 1 baseline constructed using all reference samples | 2.5 | normal | 4 | amplification | 6.1 | amplification |
| EGFR | baseline 1 (method of the present application) | 2.7 | amplification | 4.4 | amplification | 7.1 | amplification |
| | baseline 2 | 2.5 | normal | 4.2 | amplification | 6.8 | amplification |
| | baseline 3 | 2.5 | normal | 4.2 | amplification | 6.6 | amplification |
| | baseline 4 | 3.1 | normal | 5.2 | amplification | 8.2 | amplification |
| | baseline 5 | 2.5 | normal | 4.1 | amplification | 6.7 | amplification |
| | 1 baseline constructed using all reference samples | 3.1 | normal | 5.2 | amplification | 8.2 | amplification |
| FGFR1 | baseline 1 (method of the present application) | 2.9 | amplification | 4.8 | amplification | 7.6 | amplification |
| | baseline 2 | 2.8 | normal | 4.6 | amplification | 7.2 | amplification |
| | baseline 3 | 2.9 | normal | 4.7 | amplification | 7.5 | amplification |
| | baseline 4 | 3.1 | normal | 5.1 | amplification | 8 | amplification |
| | baseline 5 | 2.7 | normal | 4.5 | amplification | 7.2 | amplification |
| | 1 baseline constructed using all reference samples | 3.5 | amplification | 5.8 | amplification | 9.1 | amplification |

(continued)

| gene | baseline | standard sample 1 | | standard sample 2 | | standard sample 3 | |
|---|---|---|---|---|---|---|---|
| | | copy number detected | detection result | copy number detected | detection result | copy number detected | detection result |
| MET | baseline 1 (method of the present application) | 2.9 | amplification | 4.6 | amplification | 7.3 | amplification |
| | baseline 2 | 3.2 | amplification | 5.1 | amplification | 8.1 | amplification |
| | baseline 3 | 2.8 | normal | 4.4 | amplification | 7 | amplification |
| | baseline 4 | 3.2 | amplification | 5.2 | amplification | 8.1 | amplification |
| | baseline 5 | 2.9 | amplification | 4.6 | amplification | 7.2 | amplification |
| | 1 baseline constructed using all reference samples | 2.9 | normal | 4.6 | amplification | 7.3 | amplification |

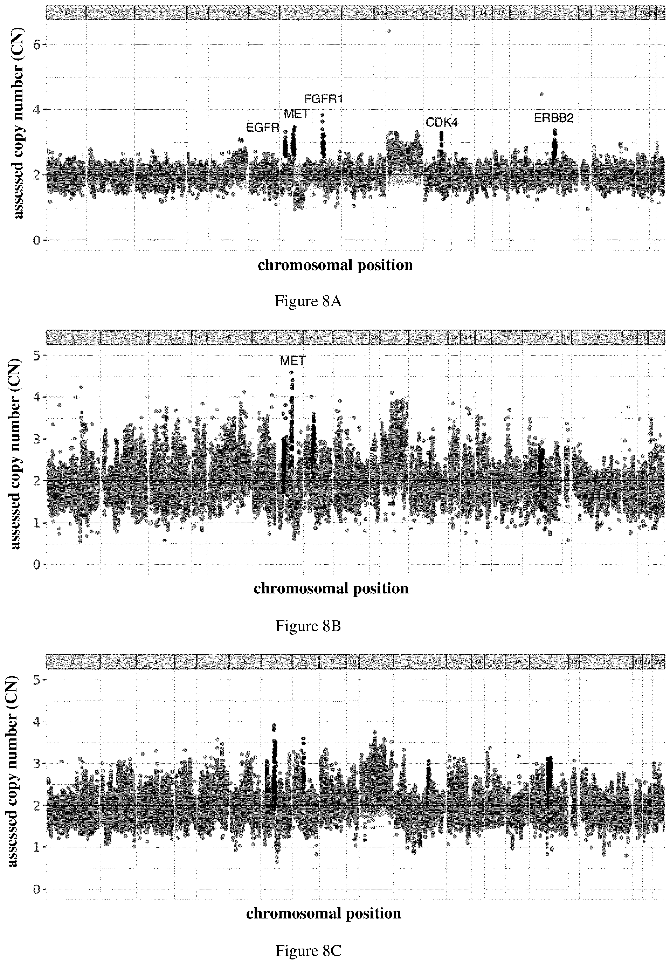

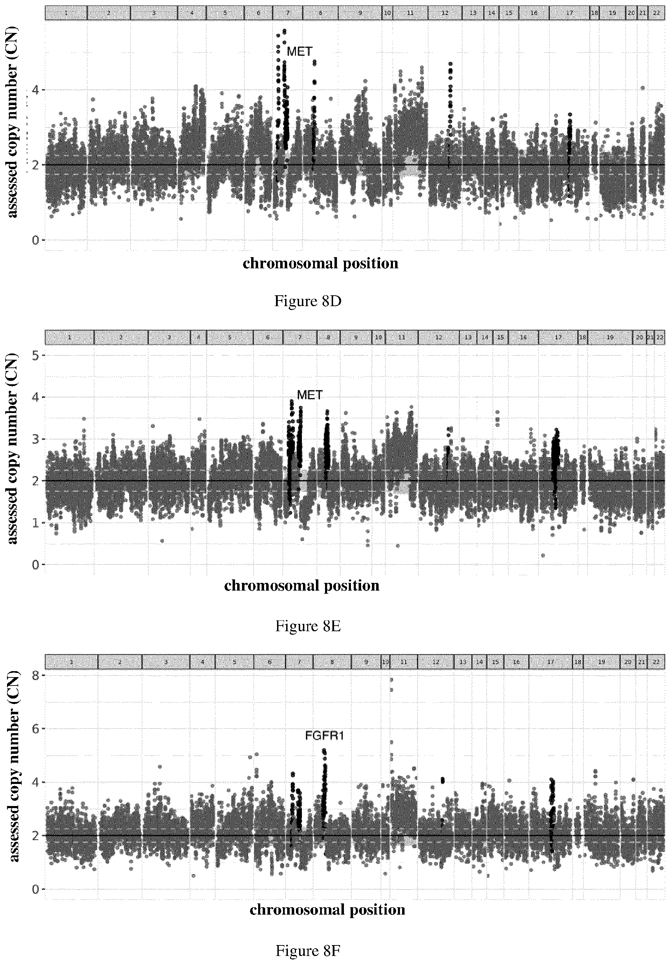

Figures 8A-8F showed examples of copy number distribution for Standard Sample 1 using different baselines.

The results indicated that the optimal baseline matched using the method of the present application is closest to the sample to be tested (the distance value between the sample and the baseline is the smallest), resulting in the lowest overall fluctuation in copy number (SD), the most stable copy number distribution graph, and minimal noise, indicating that the detection results of this method were more stable. The method of present application could stably detect all genes and different copy number states, whereas other baselines could not stably detect when the copy number was 3.

The detailed description provided above is for explanation and example purposes and is not intended to limit the scope of the claims attached. Various modifications of the embodiments listed in the present application are apparent to those skilled in the art and are intended to fall within the scope of the attached claims and their equivalents.

**Claims**

1. A copy number status analysis method,
   the method includes dividing a target region of a sample to be tested into several windows, acquiring sequencing data of a control window area within a sample group to be tested, and determining copy number status of a target gene of the sample to be tested based on the sequencing data of the control window region; optionally, the control window region includes window regions with low coverage fluctuation levels.

2. The method according to claim 1, the method further includes following steps:

   (S 1) acquiring sequencing data of the sample to be tested and/or sequencing data of multiple reference samples;
   (S2) dividing the reference samples into two or more reference sample groups;
   (S3) determining a reference sample group closest to the sample to be tested;
   (S4) determining copy number status of the target gene of the sample to be tested based on sequencing data of the reference sample group closest to the sample to be tested.

3. The method according to any one of claims 1-2,
   the method includes sorting window regions of quality-approved samples according to coverage fluctuation levels from low to high; the control window regions include top two or more, or top four or more window regions based on coverage fluctuation levels, or a ratio of median absolute deviation to median of the sequencing data of all the quality-approved samples in the control window regions is approximately 0.15 or smaller.

**4.** The method according to claim 3,
the method includes determining the coverage fluctuation levels based on statistical values of sequencing data from window regions of the quality-approved sample; optionally, determining the coverage fluctuation levels based on a ratio of median absolute deviation to median of sequencing data from window regions of the quality-approved sample.

**5.** The method according to any one of claims 3-4,
the method includes determining a normalization coefficient based on the sequencing data of the control window regions; optionally, determining the normalization coefficient by calculating average sequencing data of all the quality-approved samples in control window regions.

**6.** The method according to claim 5,
the method includes determining copy number of each window region of sample to be tested based on the normalization coefficient; optionally, the normalization includes dividing the sequencing data of the sample to be tested in the window region by normalization coefficient of the window region and multiplying by ploidy.

**7.** The method according to any one of claims 1-6,
the method includes determining significance of copy number variation of the sample to be tested based on sequencing data of each window region of the sample to be tested and sequencing data of other samples in the sample group to be tested for corresponding window region; optionally, determining the significance of the copy number variation by T-test.

**8.** The method according to any one of claims 1-7,
the sample to be tested is selected from the group consisting of: tissue, blood, saliva, pleural effusion, peritoneal effusion, and cerebrospinal fluid.

**9.** The method according to any one of claims 2-8,

the step (S2) includes step (S2-1): grouping the reference samples, the grouping includes grouping the reference samples by a cluster analysis method based on the sequencing data of the target regions; preferably, the cluster analysis method includes K-means clustering and/or hierarchical clustering;
the step (S2) includes step (S2-2): confirming statistical values of the sequencing data of the reference sample group; preferably, the confirming statistical values include calculating the mean and/or standard deviation of the reference samples in each group over the target regions.

**10.** The method according to any one of claims 2-9,
the step (S3) includes confirming degree of distribution similarity between the reference sample group and the sample to be tested based on the sequencing data of the reference sample group and the sample to be tested on the target regions by calculating statistical distance; preferably, a high degree of distribution similarity includes a short statistical distance between the reference sample group and the sample to be tested on the target regions.

**11.** The method according to claim 10,
the statistical distance includes statistical value of p-th power of absolute value of difference in the sequencing data of the reference sample group and the sample to be tested on the target regions, the p is 1 or greater; preferably, the statistical value includes a sum value; preferably, the statistical distance includes Minkowski distance.

**12.** The method according to any one of claims 2-11,

the step (S4) includes determining copy number $CN_g$ of the target gene on the sample to be tested based on length of exon of the target gene of the sample to be tested and copy number $CN_i$ on target region i of the sample to be tested; preferably, the step (S4) includes determining the $CN_g$ based on following formula,

$$CN_g = \frac{\sum_j^m \left(\frac{\sum_i^n (CN_i)}{n} * Len_j\right)}{\sum_j^m Len_j}$$

wherein i represents target region, j represents target exon, n represents number of target regions on target exon j, m represents number of target exons, $CN_i$ represents copy number of target region i, $Len_j$ represents

length of target exon j.

13. The method according to any one of claims 2-12,

the step (S4) includes: determining probability of copy number variation in the sample to be tested on the target region, where the probability of copy number variation includes probability ($p_a$) of copy number amplification and/or probability ($p_d$) of copy number deletion on the target region; preferably, step (S4) includes, based on the sequencing data of the sample to be tested on target region i, and mean and standard deviation of the sequencing data of closest reference sample group on corresponding target region, confirming the probability of copy number variation through a probability distribution method; preferably, the probability distribution includes a normal distribution.

14. The method according to any one of claims 2-13,

the step (S4) includes: determining proportion sigRatio of significant copy number amplification or deletion on the target gene of the sample to be tested; preferably, by dividing number of target regions with significant copy number variation on the target gene by total number of target regions on the target gene to obtain the sigRatio; the target regions with significant copy number variation include target regions where the proportion of copy number variation is approximately 30% or higher;

the step (S4) further includes: determining statistical test parameters for copy number variation on the target gene of the sample to be tested; preferably, based on the number of target regions on the target gene of the sample to be tested, the sequencing data of each target region on the target gene of the sample to be tested, standard deviation of the sequencing data of each target region on the target gene of the sample to be tested, and the mean and standard deviation of the sequencing data of the closest reference sample group on the corresponding target gene, confirming p-value $p_{ttest}$ by T-test.

15. The method according to claim 14,

the step (S4) determines copy number status of the target gene of the sample to be tested as follows:

when $CN_g \geq CN_{thA}$, sigRatio$\geq$sigRatio$_{th}$, and $p_{ttest} \leq p_{th}$, it is confirmed that the target gene of the sample to be tested has undergone copy number amplification;

when $CN_g \leq CN_{thD}$, sigRatio $\geq$ sigRatio$_{th}$, and $p_{ttest} \leq p_{th}$, it is confirmed that the target gene of the sample to be tested has undergone copy number deletion;

when $CN_{thA} < CN_g < CN_{thD}$, or sigRatio<sigRatio$_{th}$, or $p_{ttest} > p_{th}$, it is confirmed that the copy number of the target gene of the sample to be tested is normal, wherein $CN_{thA}$, $CN_{thD}$, sigRatio$_{th}$, and $p_{th}$ are independently thresholds; preferably, $CN_{thA}$ is approximately 2.25 to approximately 4; preferably, $CN_{thD}$ is approximately 1.0 to approximately 1.75; preferably, sigRatio$_{th}$ is approximately 0.3 to approximately 1; preferably, $p_{th}$ is approximately 0.05 to approximately 0.00001.

16. The method according to any one of claims 2-15,

the target gene includes genes selected from following group: ABL1, ABL2, ABRAXAS1, ACVR1, ACVR1B, AKT1, AKT2, AKT3, ALK, ALOX12B, AMER1, APC, AR, ARAF, ARFRP1, ARID1A, ARID1B, ARID2, ARID5B, ASXL1, ASXL2, ASXL3, ATG5, ATM, ATR, ATRX, AURKA, AURKB, AXIN1, AXIN2, AXL, B2M, BAP1, BARD1, BBC3, BCL10, BCL2, BCL2L1, BCL2L11, BCL2L2, BCL6, BCOR, BCORL1, BIRC3, BLM, BMPR1A, BRAF, BRCA1, BRCA2, BRD4, BRD7, BRINP3, BRIP1, BTG1, BTG2, BTK, CALR, CARD11, CASP8, CBFB, CBL, CCND1, CCND2, CCND3, CCNE1, CD274, CD28, CD58, CD74, CD79A, CD79B, CDC73, CDH1, CDH18, CDK12, CDK4, CDK6, CDK8, CDKN1A, CDKN1B, CDKN1C, CDKN2A, CDKN2B, CDKN2C, CEBPA, CENPA, CHD1, CHD2, CHD4, CHD8, CHEK1, CHEK2, CIC, CIITA, CREBBP, CRKL, CRLF2, CRYBG1, CSF1R, CSF3R, CSMD1, CSMD3, CTCF, CTLA4, CTNNA1, CTNNB1, CUL3, CUL4A, CXCR4, CYLD, CYP17A1, CYP2D6, DAXX, DCUN1D1, DDR1, DDR2, DDX3X, DICER1, DIS3, DNAJB1, DNMT1, DNMT3A, DNMT3B, DOT1L, DPYD, DTX1, DUSP22, EED, EGFR, EIF1AX, EIF4E, EMSY, EP300, EPCAM, EPHA2, EPHA3, EPHA5, EPHA7, EPHB1, EPHB4, ERBB2, ERBB3, ERBB4, ERCC1, ERCC2, ERCC3, ERCC4, ERCC5, ERG, ERRFI1, ESR1, ETV4, ETV5, ETV6, EWSR1, EZH2, EZR, FANCA, FANCC, FANCD2, FANCE, FANCF, FANCG, FANCI, FANCL, FANCM, FAS, FAT1, FAT3, FBXW7, FGF10, FGF12, FGF14, FGF19, FGF23, FGF3, FGF4, FGF6, FGF7, FGFR1, FGFR2, FGFR3, FGFR4, FH, FLCN, FLT1, FLT3, FLT4, FOXA1, FOXL2, FOXO1, FOXO3, FOXP1, FRS2, FUBP1, FYN, GABRA6, GALNT12, GATA1, GATA2, GATA3, GATA4, GATA6, GEN1, GID4, GLI1, GNA11, GNA13, GNAQ, GNAS, GPS2, GREM1, GRIN2A, GRM3, GSK3B, H3F3A, H3F3B, H3F3C, HDAC1, HDAC2, HGF, HIST1H1C, HIST1H2BD, HIST1H3A, HIST1H3B, HIST1H3C, HIST1H3D, HIST1H3E, HIST1H3G, HIST1H3H, HIST1H3I, HIST1H3J, HIST2H3D, HIST3H3, HLA-A, HLA-B, HLA-C, HNF1A, HOXB13, HRAS, HSD3B1, HSP90AA1, ICOSLG, ID3, IDH1, IDH2, IFNGR1, IGF1, IGF1R, IGF2, IGHD, IGHJ, IGHV, IKBKE, IKZF1, IL10, IL7R, INHA, INHBA, INPP4A, INPP4B, INSR, IRF2, IRF4, IRS1, IRS2, ITK, ITPKB, JAK1, JAK2, JAK3, JUN, KAT6A, KDM5A, KDM5C, KDM6A, KDR, KEAP1, KEL, KIR2DL4, KIR3DL2, KIT, KLF4, KLHL6, KLRC1, KLRC2, KLRK1, KMT2A, KMT2C, KMT2D, KRAS, LATS1, LATS2, LMO1, LRP1B, LTK, LYN, MAF, MAGI2, MALT1, MAP2K1, MAP2K2, MAP2K4, MAP3K1, MAP3K13, MAP3K14, MAPK1, MAPK3, MAX, MCL1, MDC1, MDM2, MDM4, MED12, MEF2B, MEN1, MERTK, MET, MFHAS1, MGA, MIR21, MITF, MKNK1, MLH1, MLH3, MPL, MRE11, MSH2, MSH3, MSH6, MST1, MST1R, MTAP, MTOR, MUTYH, MYC, MYCL, MYCN, MYD88, MYOD1, NAV3, NBN, NCOA3, NCOR1, NCOR2, NEGR1, NF1, NF2, NFE2L2, NFKBIA, NKX2-1, NKX3-1, NOTCH1, NOTCH2, NOTCH3, NOTCH4, NPM1, NRAS, NRG1, NSD1, NSD2, NSD3, NT5C2, NTHL1, NTRK1, NTRK2, NTRK3, NUP93, NUTM1, P2RY8, PAK1, PAK3, PAK5, PALB2, PALLD, PARP1, PARP2, PARP3, PAX5, PBRM1, PCDH11X, PDCD1, PDCD1LG2, PDGFRA, PDGFRB, PDK1, PGR, PHOX2B, PIK3C2B, PIK3C2G, PIK3C3, PIK3CA, PIK3CB, PIK3CD, PIK3CG, PIK3R1, PIK3R2, PIK3R3, PIM1, PLCG2, PLK2, PMS1, PMS2, PNRC1, POLD1, POLE, POM121L12, PPARG, PPM1D, PPP2R1A, PPP2R2A, PPP6C, PRDM1, PREX2, PRKAR1A, PRKCI, PRKDC, PRKN, PTCH1, PTEN, PTPN11, PTPRD, PTPRO, PTPRS, PTPRT, QKI, RAB35, RAC1, RAD21, RAD50, RAD51, RAD51B, RAD51C, RAD51D, RAD52, RAD54L, RAF1, RARA, RASA1, RB1, RBM10, RECQL4, REL, RET, RHEB, RHOA, RICTOR, RIT1, RNF43, ROS1, RPA1, RPS6KA4, RPS6KB2, RPTOR, RSP02, RUNX1, RUNX1T1, SDC4, SDHA, SDHAF2, SDHB, SDHC, SDHD, SETD2, SF3B 1, SGK1, SH2B3, SH2D1A, SHQ1, SLC34A2, SLIT2, SLX4, SMAD2, SMAD3, SMAD4, SMARCA4, SMARCB1, SMARCD1, SMO, SNCAIP, SOCS1, SOX10, SOX17, SOX2, SOX9, SPEN, SPI1, SPOP, SPTA1, SRC, SRSF2, STAG2, STAT3, STAT4, STAT5A, STAT5B, STAT6, STK11, STK40, SUFU, SYK, TAF1, TBX21, TBX3, TCF3, TCF7L2, TEK, TENT5C, TERC, TERT, TET1, TET2, TGFBR1, TGFBR2, TIPARP, TMEM127, TMPRSS2, TNFAIP3, TNFRSF14, TOP1, TOP2A, TP53, TP63, TP73, TRAF2, TRAF3, TRAF7, TRIM58, TRPC5, TSC1, TSC2, TSHR, TYRO3, U2AF1, UGT1A1, VEGFA, VEGFB, VEGFC, VHL, WISP3, WRN, WT1, XIAP, XPO1, XRCC2, XRCC3, YAP1, YES1, ZAP70, ZBTB16, ZBTB2, ZNF217, ZNF703 and ZNRF3.

17. A copy number status analysis device, including following modules: a receiving module for acquiring sequencing data of a sample group to be tested; a determination module for determining the target gene of the sample to be tested; a judgment module for determining the copy number status of the target gene of the sample to be tested based on the sequencing data of the sample group to be tested.

18. The copy number status analysis device according to claim 17, including following modules: (M1) a receiving module for acquiring sequencing data of the sample to be tested and/or sequencing data of multiple reference samples; (M2) a processing module for dividing the reference samples into two or more reference sample groups; (M3) a computational module for determining the reference sample group closest to the sample to be tested; (M4) a judgment module for determining the copy number status of the target gene of the sample to be tested based on the sequencing data of the reference sample group closest to the sample to be tested.

19. A storage medium, which records a program capable of executing the method according to any one of claims 1-16.

**Figure 1A**

**Figure 1B**

**Figure 2A**

**Figure 2B**

Figure 3A

Figure 3B

BRCA1 exon 16-19 copy number amplification

Figure 4A

BRCA1 exon 3-7 copy number amplification

Figure 4B

BRCA2 exon 14-18 copy number amplification

Figure 4C

BRCA2 exon 3 copy number amplification

Figure 4D

BRCA2 exon 25-27 copy number amplification

Figure 4E

BRCA1 exon 15-22 copy number deletion

Figure 4F

BRCA1 exon 2 copy number deletion

Figure 4G

BRCA1 exon 7-12 copy number deletion

Figure 4H

BRCA2 exon 15-16 copy number deletion

Figure 4I

BRCA2 exon 8-11 copy number deletion

Figure 4J

Figure 5A

Figure 5B

Figure 5C

Figure 5D

Figure 5E

Figure 5F

Figure 6

Figure 7

Figure 8A

Figure 8B

Figure 8C

Figure 8D

Figure 8E

Figure 8F

## INTERNATIONAL SEARCH REPORT

International application No.

**PCT/CN2022/115447**

### A. CLASSIFICATION OF SUBJECT MATTER

C12Q 1/6869(2018.01)i; G16B 20/20(2019.01)i; G16B 20/30(2019.01)i; G16B 30/10(2019.01)i

According to International Patent Classification (IPC) or to both national classification and IPC

### B. FIELDS SEARCHED

Minimum documentation searched (classification system followed by classification symbols)

C12Q; G16B

Documentation searched other than minimum documentation to the extent that such documents are included in the fields searched

Electronic data base consulted during the international search (name of data base and, where practicable, search terms used)

CNTXT, CNABS, DWPI, WPABS, VEN, ENTXT, USTXT, CNKI, CJFD, IEEE: 拷贝数, 变异, 基因, 扩增, 缺失, 窗口, 均值, 标准差, 中位数, 距离, 相似度, 测序数据, 显著性, 检验, 校正, 矫正, 比对, 对照, 比较, 概率, 比例, 区间, 归一化, Copy number, Variation, gene, amplificat+, delet+, window?, mean, standard deviation, median, distance, similarity, sequencing data, significance, test+, correct+, alignment, control+, compar+, probability, proportion, interval, Normalizat+

### C. DOCUMENTS CONSIDERED TO BE RELEVANT

| Category* | Citation of document, with indication, where appropriate, of the relevant passages | Relevant to claim No. |
|---|---|---|
| PX | CN 113789371 A (GUANGZHOU BURNING ROCK MEDICAL EXAMINATION INSTITUTE CO., LTD.) 14 December 2021 (2021-12-14) paragraphs 45-85 | 1, 3-7, 19 |
| PX | CN 113674803 A (GUANGZHOU BURNING ROCK MEDICAL EXAMINATION INSTITUTE CO., LTD.) 19 November 2021 (2021-11-19) claim 8, and paragraphs 163-168 | 17-18 |
| X | CN 108256292 A (ANNOROAD GENE TECHNOLOGY (BEIJING) CO., LTD. et al.) 06 July 2018 (2018-07-06) paragraphs 50-112 | 1-19 |
| X | CN 106650312 A (ANNOROAD GENE TECHNOLOGY (BEIJING) CO., LTD.) 10 May 2017 (2017-05-10) paragraphs 7-59 | 1-19 |
| X | US 2013316915 A1 (HALPERN AARON et al.) 28 November 2013 (2013-11-28) paragraphs 45-274 | 1-19 |

☑ Further documents are listed in the continuation of Box C.    ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 October 2022** | **25 November 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

# EP 4 397 773 A1

## INTERNATIONAL SEARCH REPORT

| | International application No. |
|---|---|
| | **PCT/CN2022/115447** |

**C.    DOCUMENTS CONSIDERED TO BE RELEVANT**

| Category* | Citation of document, with indication, where appropriate, of the relevant passages | Relevant to claim No. |
|---|---|---|
| X | CN 111968701 A (BEIJING GENE+ TECHNOLOGY CO., LTD. et al.) 20 November 2020 (2020-11-20)<br>    paragraphs 53-71 | 17-18 |
| Y | CN 113789371 A (GUANGZHOU BURNING ROCK MEDICAL EXAMINATION INSTITUTE CO., LTD.) 14 December 2021 (2021-12-14)<br>    paragraphs 45-85 | 2, 8-16, 19 |
| Y | CN 113674803 A (GUANGZHOU BURNING ROCK MEDICAL EXAMINATION INSTITUTE CO., LTD.) 19 November 2021 (2021-11-19)<br>    claim 8, and paragraphs 163-168 | 2, 8-16, 19 |
| A | CN 106951737 A (SOUTHERN MEDICAL UNIVERSITY) 14 July 2017 (2017-07-14)<br>    entire document | 1-19 |
| A | CN 108427864 A (GENESEEQ TECHNOLOGY INC.) 21 August 2018 (2018-08-21)<br>    entire document | 1-19 |
| A | CN 104133914 A (XIAMEN VANGENES BIOTECHNOLOGY CO., LTD.) 05 November 2014 (2014-11-05)<br>    entire document | 1-19 |
| A | CN 106715711 A (SHENZHEN BGI GENOMICS CO., LTD.) 24 May 2017 (2017-05-24)<br>    entire document | 1-19 |
| A | US 2016300013 A1 (AGILENT TECHNOLOGIES INC.) 13 October 2016 (2016-10-13)<br>    entire document | 1-19 |

Form PCT/ISA/210 (second sheet) (January 2015)

<div align="center">

**INTERNATIONAL SEARCH REPORT**
**Information on patent family members**

</div>

International application No.

**PCT/CN2022/115447**

| Patent document cited in search report | | | Publication date (day/month/year) | Patent family member(s) | | | Publication date (day/month/year) |
|---|---|---|---|---|---|---|---|
| CN | 113789371 | A | 14 December 2021 | | None | | |
| CN | 113674803 | A | 19 November 2021 | | None | | |
| CN | 108256292 | A | 06 July 2018 | CN | 108256292 | B | 02 November 2021 |
| CN | 106650312 | A | 10 May 2017 | CN | 106650312 | B | 17 May 2022 |
| US | 2013316915 | A1 | 28 November 2013 | US | 2016117444 | A1 | 28 April 2016 |
| CN | 111968701 | A | 20 November 2020 | | None | | |
| CN | 106951737 | A | 14 July 2017 | CN | 106951737 | B | 14 June 2019 |
| CN | 108427864 | A | 21 August 2018 | WO | 2019157791 | A1 | 22 August 2019 |
| | | | | CN | 108427864 | B | 29 January 2019 |
| CN | 104133914 | A | 05 November 2014 | CN | 104133914 | B | 08 March 2017 |
| CN | 106715711 | A | 24 May 2017 | WO | 2016000267 | A1 | 07 January 2016 |
| | | | | CN | 106715711 | B | 17 September 2021 |
| US | 2016300013 | A1 | 13 October 2016 | US | 2021174901 | A1 | 10 June 2021 |

Form PCT/ISA/210 (patent family annex) (January 2015)